# EUROPEAN PATENT SPECIFICATION

(11) **EP 3 036 542 B1**
(45) Date of publication and mention of the grant of the patent: **17.02.2021**
(21) Application number: 14814585.7
(22) Date of filing: 21.06.2014
(51) Int. Cl.: G01N 33/48, G01N 21/59, G01N 21/3577, G01N 33/86, A61B 5/1455, A61B 5/145, C12Q 1/68

(54) **A METHOD FOR ASSAYING A BLOOD SAMPLE OBTAINED FROM A SUBJECT**
EINE METHODE ZUR UNTERSUCHUNG EINER VON EINER PERSON ERHALTENEN BLUTPROBE
PROCÉDÉ DE DOSAGE D'UN ÉCHANTILLON DE SANG OBTENU À PARTIR D'UN SUJET

(30) Priority: 21.06.2013 US 201361838113 P
(43) Date of publication of application: 29.06.2016
(73) Proprietor: Imigene Inc., Saint Petersburg, FL 33716 (US)
(72) Inventor: EWERT, Matt, St. Petersburg, FL 33703 (US)
(74) Representative: Gill Jennings & Every LLP
(86) International application number: PCT/US2014/043540
(87) International publication number: WO 2014/205426

(56) References cited:
- US-A- 5 789 387
- US-A1- 2008 268 483
- US-A1- 2011 059 460
- US-A1- 2011 059 460
- US-A1- 2012 045 826
- US-A1- 2012 173 154
- US-A1- 2012 190 834
- US-B1- 6 855 562
- ALEKSANDRA PALUSZKIEWICZ ET AL: "Effect of Hemolysis and Free Hemoglobin on Optical Hematocrit Measurements in the Extracorporeal Circulation", ASAIO JOURNAL, vol. 54, no. 2, 1 March 2008 (2008-03-01), pages 181-184, XP055160969, ISSN: 1058-2916, DOI: 10.1097/MAT.0b013e318164dcc6
- GUREVICH ET AL: "Determination of Fc function with frozen red blood cells", BIOLOGICALS, ACADEMIC PRESS LTD., LONDON, GB, vol. 34, no. 3, 1 September 2006 (2006-09-01), pages 221-222, XP005598586, ISSN: 1045-1056, DOI: 10.1016/J.BIOLOGICALS.2005.11.003
- WEINSTEIN R E ET AL: "Species specificity of the fibrinolytic effects of activated protein C", THROMBOSIS RESEARCH, TARRYTOWN, NY, US, vol. 63, no. 1, 1 July 1991 (1991-07-01), pages 123-131, XP026398059, ISSN: 0049-3848 [retrieved on 1991-07-01]

## Description

### CROSS-REFERENCE TO RELATED APPLICATIONS

This application claims priority from U.S. Provisional Application No. 61/838,113, entitled "BLOOD ANALYSIS", filed June 21, 2013.

### BACKGROUND OF THE INVENTION

Bacterial infections are associated with haemostatic alterations that range from hypercoagulability to acute disseminated intravascular coagulation (DIC). Infection related disseminated intravascular coagulation involves activation of the procoagulant pathway resulting in micro thrombosis and organ failure (1-2). DIC occurs in approximately 30% of patients with severe sepsis (3). Existing diagnostic tests for DIC are hindered by specificity and sensitivity levels that do not significantly impact care (4-6).

Many biomarkers for infection and sepsis have been described. The D-dimer assay is based on detection of a fibrin degradation product. One recent study suggests that high levels of D-dimer are associated with 28-day mortality in patients with infection or sepsis identified in the emergency department (8). Diagnostic utility for the D-dimer test lies in its negative predictive value with high sensitivity but low specificity. Procalcitonin (PCT) has been shown to be useful for early diagnosis of sepsis in critically ill patients when results are included in the context of medical history, physical examination, and microbiological assessment (9). The widespread study of PCT was preceded by evaluations for C-reactive protein (CRP) utility. CRP was found to poorly distinguish sepsis from Systemic Inflammatory Response Syndrome (SIRS) without infection. In a recent adult emergency department study involving suspected cases of sepsis, PCT, IL-6, and CRP highly correlated with several infection parameters but were all found to be inadequately discriminating for use as an independent front line diagnostic tool (10). These biomarker methods are based on the use of plasma as the sample.

Thromboelastometry or TEM (known previously as rotational thrombelastometry) is an established method for hemostasis testing in whole blood. TEM evaluates interactions between coagulation factors/inhibitors, anticoagulant drugs, blood cells and platelets during clotting and subsequent fibrinolysis. The assay is conducted in a vessel that mimics rheological conditions found in blood vessels. TEM represents an enhancement of the thrombelastography method originally described by H. Hartert in 1948 (7). TEM is a reliable rapid test for the diagnosis of hyper fibrinolysis based on the impedance of a pin suspended. The primary result of TEM is a reaction curve which shows the elasticity over time when the clot forms or dissolves in the reaction vessel. Preservation of red blood cell integrity is essential for this method to function.

A recent advance in the diagnosis of infection based on an assay in blood is described in U.S. Publication 20110059460. In this published application, as assay is described involving measurement of the rate at which foam dissolves, after frozen blood has been shaken.

### SUMMARY OF THE INVENTION

Aspects of the invention are based, in part, on the unexpected finding that the transmittance of polarized light over multiple wavelengths through the various differently-processed blood samples (e.g., with plasmin or phospholipase) after a freeze-thaw cycle identified not only the presence or absence of infection in a subject, but also the type of infection (e.g., blood stream infection, urinary tract infection, bacterial pneumonia, lower respiratory tract infection, or systemic viral infection).

Aspects of the invention involve the discovery that blood has different characteristics depending on whether the blood is from an infected person or an uninfected person. These characteristics can be revealed by observing the ability of blood to transmit light under various conditions. In one aspect, the observation can be enhanced by treating the blood with different processing steps, such as freezing and thawing the blood and/or treating the blood with enzymes. In another aspect, the observation can be enhanced by measuring the ability of blood to transmit light at different wave-lengths. In another aspect, the observation can be enhanced by comparing light transmittance under two or more conditions to one another, at the same wavelength or across a number of wavelengths. (In the description below, when it is said compare light transmittance of the light through a first blood sample with the transmittance of light through a second blood sample, it is typically meant compare a value obtained for light transmittance through the first sample to a value obtained for light transmittance through the second sample.) Light transmittance of each sample is typically carried out at the same wavelength and at the same polarization. The comparison can be determining relative values such as one being higher or lower than the other, adding values together, subtracting values from one another, multiplying values, and the like. As will be seen below, patterns characteristic not only of the existence of infection, but specific to particular infections (or even specific sites of infection) can be revealed. It is believed that the blood is affected in specific ways by specific infections, which specificity can be reflected in the ability of blood to transmit light under different conditions.

Some aspects of the invention relate to a method for assaying a blood sample obtained from a subject.

In some embodiments, the method further comprises comparing the transmittance of the light through the first anticoagulant-treated blood sample with the transmittance of light through the second and third anticoagulant-treated blood samples. In some embodiments, the method further comprises subtracting the transmittance of light through the second anticoagulant-treated blood sample from the transmittance of light through the first anticoagulant-treated blood sample and subtracting the transmittance of light through the third anticoagulant-treated blood sample from the transmittance of light through the first anticoagulant-treated blood sample. In some embodiments, the method further comprises subtracting the transmittance of light through the third anticoagulant-treated blood sample from the transmittance of light through the second anticoagulant-treated blood sample. In some embodiments, the light is at a wavelength of between 775-795 nm. In some embodiments, the light is at a wavelength of between 680-700 nm.

In some embodiments of any one of the methods provided herein, the light is at a first and a second wavelength. In some embodiments, the first wavelength is between 775-795 nm and the second wavelength is between 680-700 nm. In some embodiments, the first wavelength is 785 nm and the second wavelength is 690 nm. In some embodiments, the method further comprises comparing the transmittance of the light at the first wavelength and the second wavelength through the first anticoagulant-treated blood sample with the transmittance of light at the first wavelength and the second wavelength through the second and third anticoagulant-treated blood samples. In some embodiments, the method further comprises subtracting the transmittance of light at each wavelength through the second anticoagulant-treated blood sample from the transmittance of light at each wavelength through the first anticoagulant-treated blood sample and subtracting the transmittance of light at each wavelength through the third anticoagulant-treated blood sample from the transmittance of light at each wavelength through the first anticoagulant-treated blood sample. In some embodiments, the method further comprises subtracting the transmittance of light at each wavelength through the third anticoagulant-treated blood sample from the transmittance of light at each wavelength through the second anticoagulant-treated blood sample.

In some embodiments of any one of the methods provided herein, the light is polarized light. In some embodiments of any one of the methods provided herein, the artificial source is a laser. In some embodiments of any one of the methods provided herein, the light is measured using a photodiode power sensor. In some embodiments, the light is measured using two photodiode power sensors and the light is passed through a bifurcated cable system prior to reaching the two photodiode power sensors.

In some embodiments of any one of the methods provided herein, the enzyme that will break down a phospholipid bilayer is phospholipase A₂. In some embodiments of any one of the methods provided herein, the plasminogen activator is streptokinase and/or urokinase. In some embodiments of any one of the methods provided herein, contacting with the plasminogen and the plasminogen activator comprises contacting with plasminogen, streptokinase, and urokinase. In some embodiments of any one of the methods provided herein, contacting with the plasmin comprises producing plasmin by combining plasminogen, streptokinase, and urokinase and contacting the sample with the produced plasmin. In some embodiments of any one of the methods provided herein, the first sample is contacted with water. In some embodiments of any one of the methods provided herein, the subject may be an animal, a mammal, a dog, a cat, a pig, a goat, a horse, a cow, or a human.

In any of the foregoing embodiments, the light may be at a wavelength of between 775-795 nm. In any of the foregoing embodiments, the light may be at a wavelength of between 680-700 nm. In some embodiments, the method of any one of the preceding claims, the light is at a first and a second wavelength, which are different. In some embodiments the first wavelength is between 775-795 nm and the second wavelength is between 680-700 nm. In any of the foregoing embodiments, the light may be polarized light. In any of the foregoing embodiments, the artificial source of light may be a laser. In any of the foregoing embodiments, the light may be measured using a photodiode power sensor. In any of the foregoing embodiments, the enzyme that breaks down phospholipids can be Phospholipase A2. In any of the foregoing embodiments, the plasminogen activator is streptokinase and/or urokinase. In any of the foregoing embodiments, contacting with the plasminogen and the plasminogen activator comprises contacting with plasminogen, streptokinase, and urokinase. In any of the foregoing embodiments, contacting with the plasmin comprising producing plasmin by combining plasminogen, streptokinase, and urokinase and contacting the sample with the produced plasmin. In any of the foregoing embodiments, the subject may be an animal, a mammal, a dog, a cat, a pig, a goat, a horse, a cow, or a human.

These and other aspects of the invention are described below.

### BRIEF DESCRIPTION OF THE DRAWINGS

The following drawings form part of the present specification and are included to further demonstrate certain aspects of the present disclosure, which can be better understood by reference to one or more of these drawings in combination with the detailed description of specific embodiments presented herein.

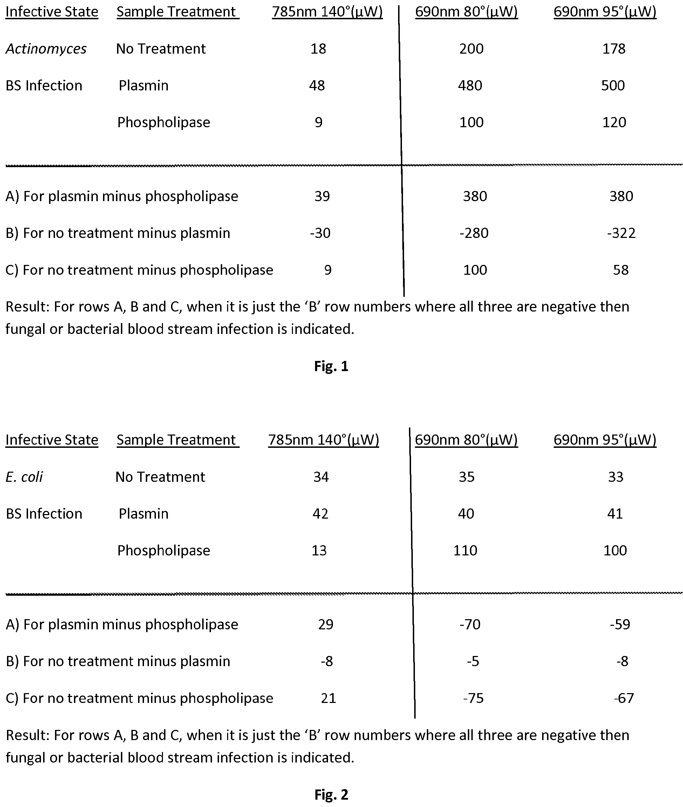

**Fig. 1** is a diagram showing light transmittance data at three different settings for three samples of whole blood obtained from an exemplary patient with *Actinomyces* blood stream (BS) infection. The samples were processed as follows: no treatment, treated with plasmin, or treated with phospholipase A₂. The values reported in the diagram are in microWatts.
**Fig. 2** is a diagram showing light transmittance data at three different settings for three samples of whole blood obtained from an exemplary patient with *E. coli* blood stream (BS) infection. The samples were processed as follows: no treatment, treated with plasmin, or treated with phospholipase A₂. The values reported in the diagram are in microWatts.

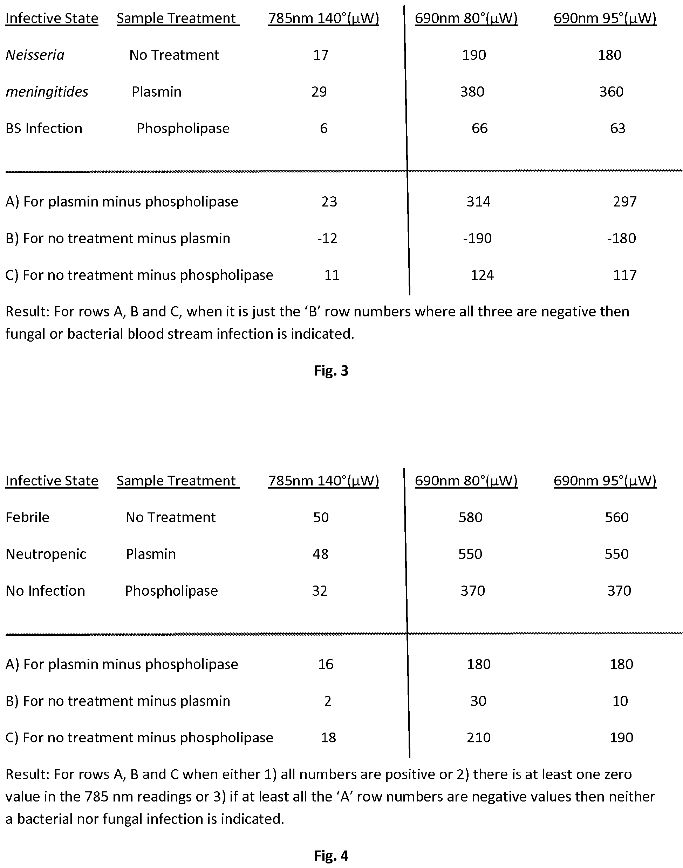

**Fig. 3** is a diagram showing light transmittance data at three different settings for three samples of whole blood obtained from an exemplary patient with *Neisseria meningitidies* blood stream (BS) infection. The samples were processed as follows: no treatment, treated with plasmin, or treated with phospholipase A₂. The values reported in the diagram are in microWatts.
**Fig. 4** is a diagram showing light transmittance data at three different settings for three samples of whole blood obtained from an exemplary patient with no infection. The samples were processed as follows: no treatment, treated with plasmin, or treated with phospholipase A₂. The values reported in the diagram are in microWatts.

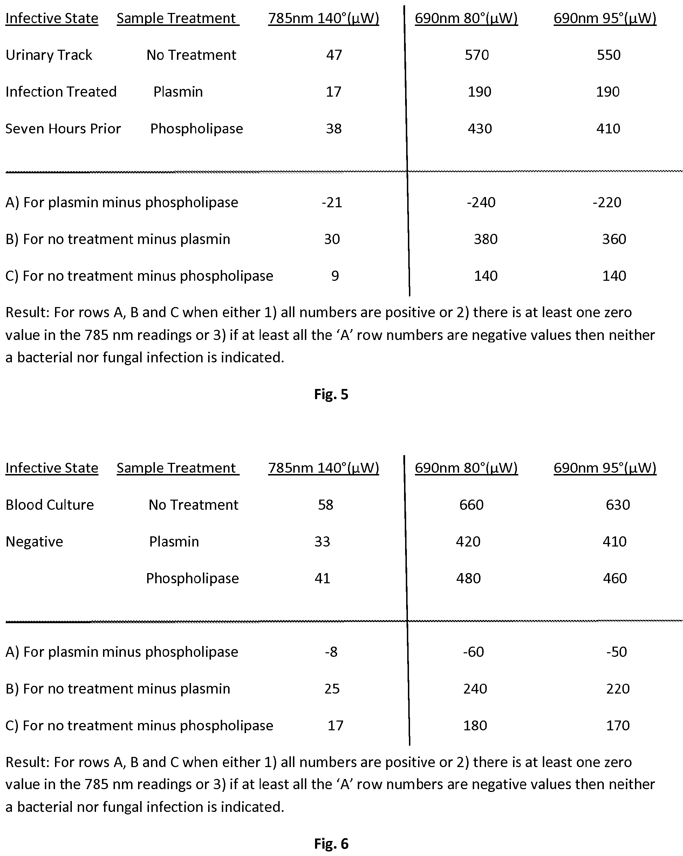

**Fig. 5** is a diagram showing light transmittance data at three different settings for three samples of whole blood obtained from an exemplary patient with a treated urinary tract infection. The samples were processed as follows: no treatment, treated with plasmin, or treated with phospholipase A₂. The values reported in the diagram are in microWatts.
**Fig. 6** is a diagram showing light transmittance data at three different settings for three samples of whole blood obtained from an exemplary patient with no infection (blood culture negative). The samples were processed as follows: no treatment, treated with plasmin, or treated with phospholipase A₂. The values reported in the diagram are in microWatts.

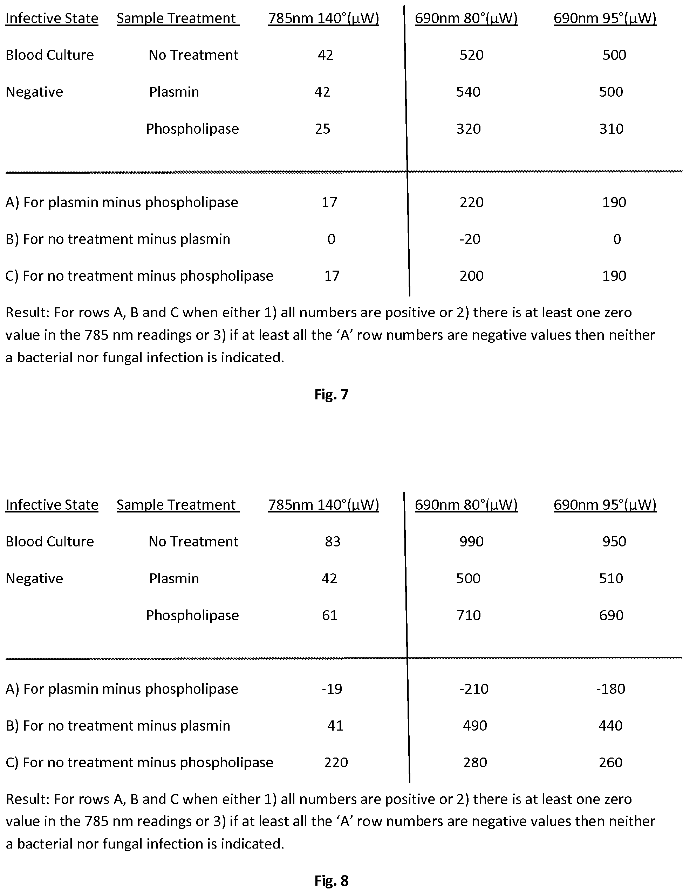

**Fig. 7** is a diagram showing light transmittance data at three different settings for three samples of whole blood obtained from an exemplary patient with no infection (blood culture negative). The samples were processed as follows: no treatment, treated with plasmin, or treated with phospholipase A₂. The values reported in the diagram are in microWatts.
**Fig. 8** is a diagram showing light transmittance data at three different settings for three samples of whole blood obtained from an exemplary patient with no infection (blood culture negative). The samples were processed as follows: no treatment, treated with plasmin, or treated with phospholipase A₂. The values reported in the diagram are in microWatts.

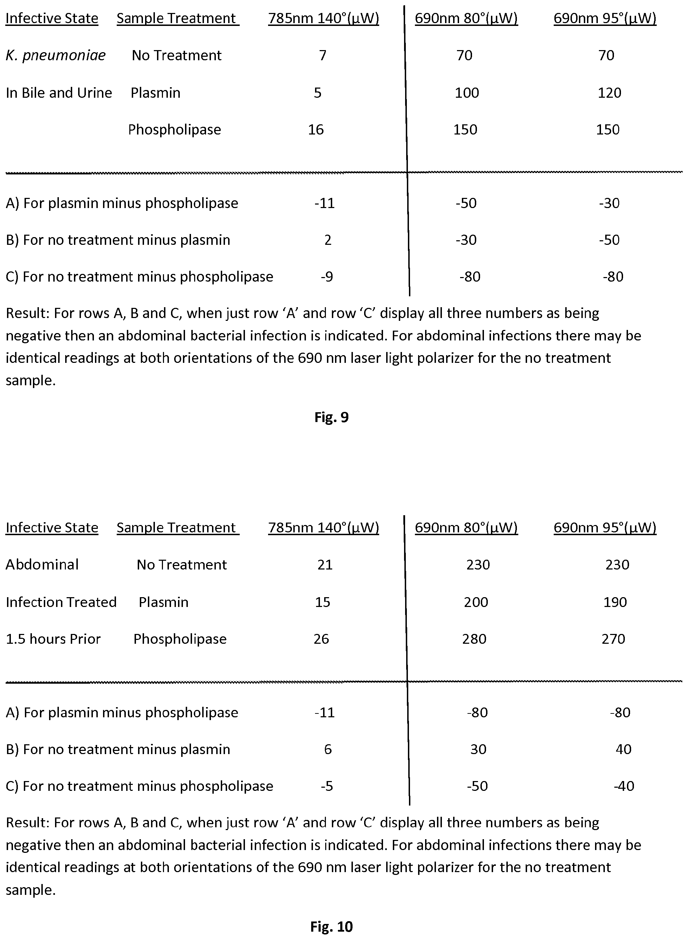

**Fig. 9** is a diagram showing light transmittance data at three different settings for three samples of whole blood obtained from an exemplary patient with *K. pneumoniae* infection in bile and urine. The samples were processed as follows: no treatment, treated with plasmin, or treated with phospholipase A₂. The values reported in the diagram are in microWatts.
**Fig. 10** is a diagram showing light transmittance data at three different settings for three samples of whole blood obtained from an exemplary patient with abdominal infection treated 1.5 hours prior. The samples were processed as follows: no treatment, treated with plasmin, or treated with phospholipase A₂. The values reported in the diagram are in microWatts.

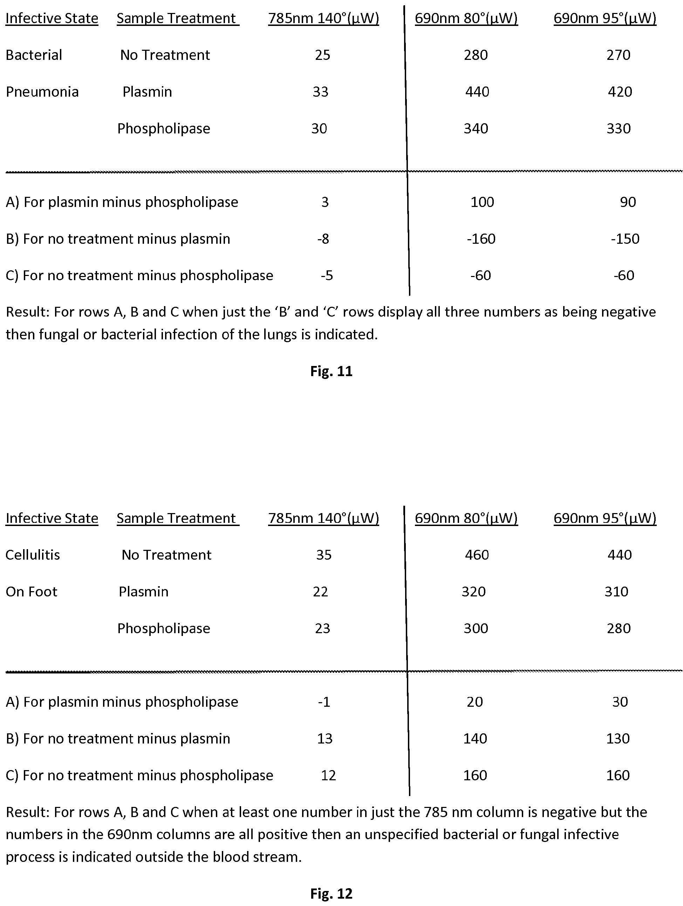

**Fig. 11** is a diagram showing light transmittance data at three different settings for three samples of whole blood obtained from an exemplary patient with bacterial pneumonia. The samples were processed as follows: no treatment, treated with plasmin, or treated with phospholipase A₂. The values reported in the diagram are in microWatts.
**Fig. 12** is a diagram showing light transmittance data at three different settings for three samples of whole blood obtained from an exemplary patient with cellulitis on foot. The samples were processed as follows: no treatment, treated with plasmin, or treated with phospholipase A₂. The values reported in the diagram are in microWatts.

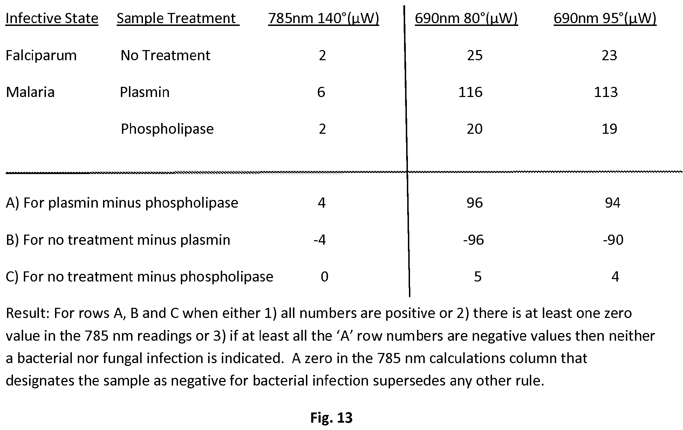

**Fig. 13** is a diagram showing light transmittance data at three different settings for three samples of whole blood obtained from an exemplary patient with Falciparum Malaria. The samples were processed as follows: no treatment, treated with plasmin, or treated with phospholipase A₂. The values reported in the diagram are in microWatts.

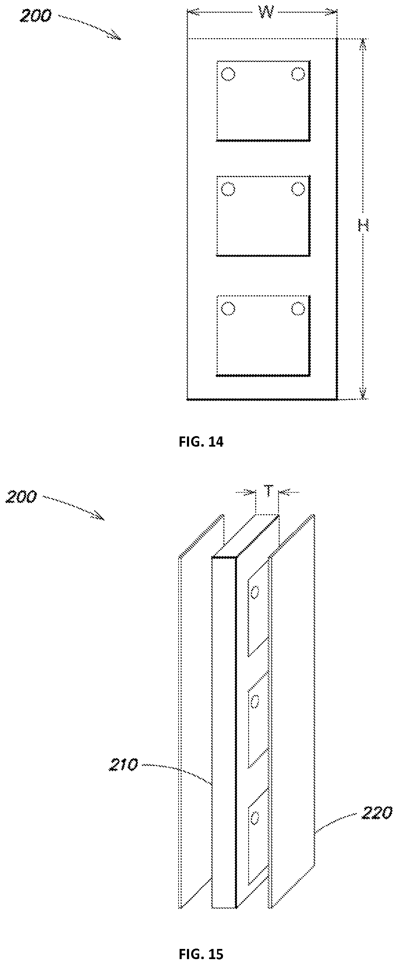

**Fig. 14** is a diagram showing an exemplary sample cartridge **200.** W = 25 mm, H = 75 mm. The holes in each square are 1 mm diameter and used to load sample into each viewing window.
**Fig. 15** is a diagram showing a different view of exemplary sample cartridge **200.** T = 5 mm. The inner part **210** is made of acrylic and the outer part **220** is made of cyclic olefin copolymer (COC).

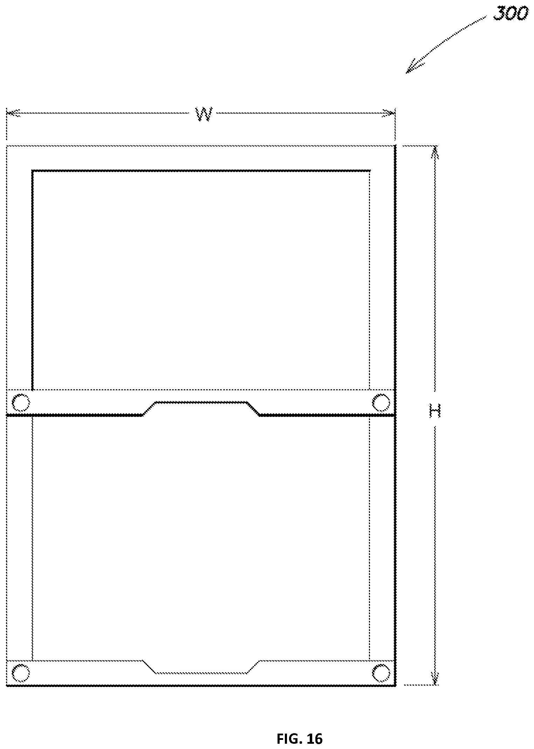

**Fig. 16** is a diagram showing an exemplary cartridge holder **300.** W = 130 mm, H = 170 mm. The cartridge is made of metal, similar to a microscope slide stage.

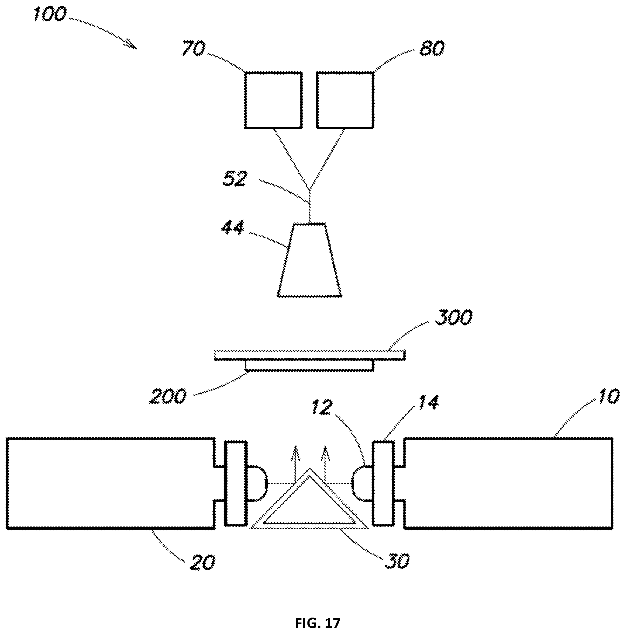

**Fig. 17** is a diagram of an exemplary polarized optical system.

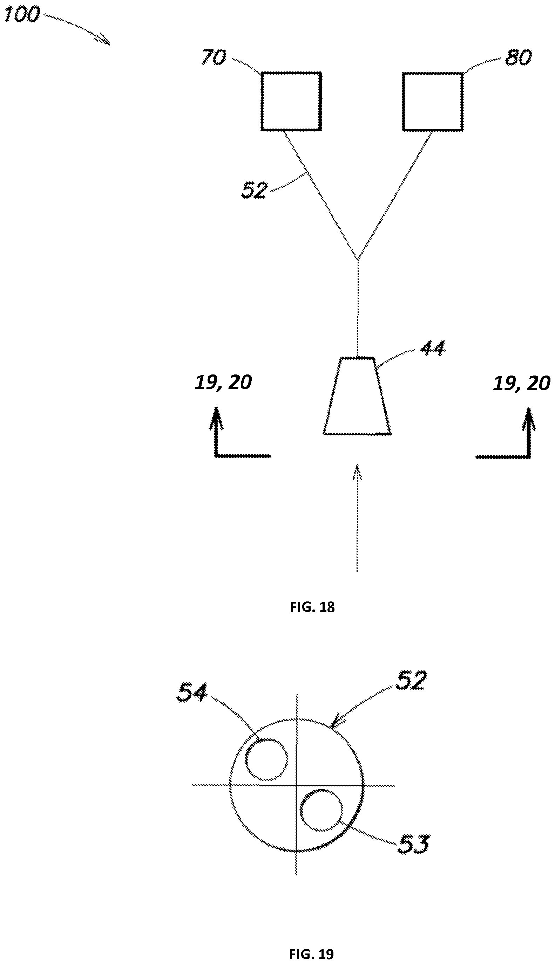

**Fig. 18** is a diagram of the bifurcated cable optical cable detection system attached to two detectors.
**Fig. 19** is a diagram of a cut-away view of the bifurcated cable showing the position of each cable (**53** and **54**) within the bifurcated cable system (**52**)**.**

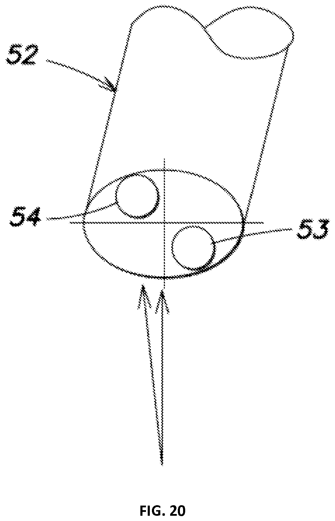

**Fig. 20** is a diagram of a cut-away view of the bifurcated cable showing the position of each cable (**53** and **54**) within the bifurcated cable system (**52**).

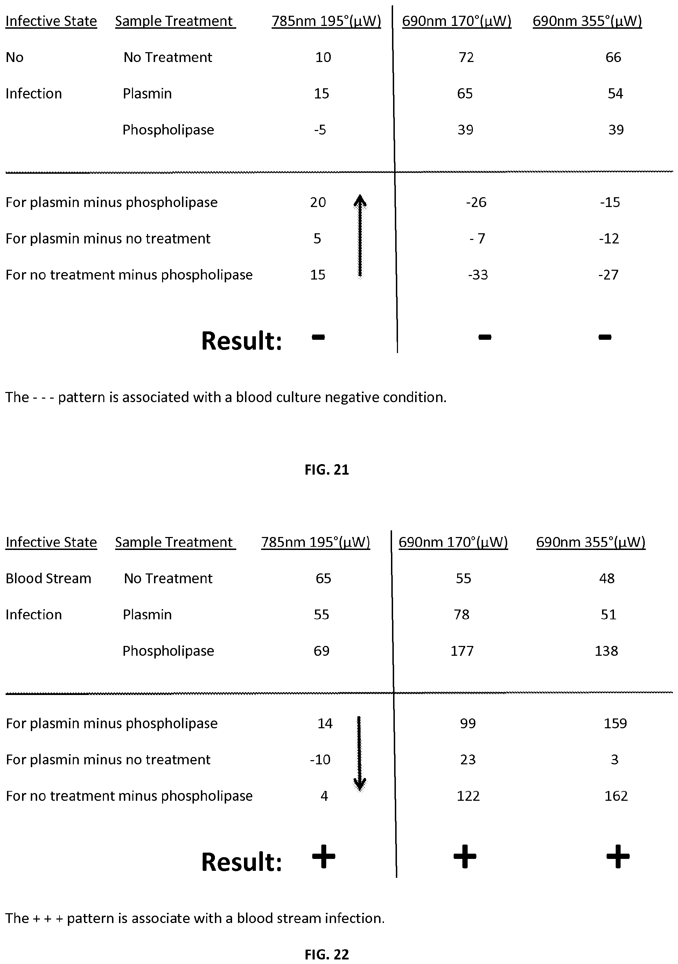

**Fig. 21** is a diagram showing light transmittance data at three different settings for three samples of whole blood obtained from an exemplary patient with no infection. The samples were processed as follows: no treatment, treated with plasmin, or treated with phospholipase A₂. The values reported in the diagram are in microWatts.
**Fig. 22** is a diagram showing light transmittance data at three different settings for three samples of whole blood obtained from an exemplary patient with a blood stream infection. The samples were processed as follows: no treatment, treated with plasmin, or treated with phospholipase A₂. The values reported in the diagram are in microWatts.

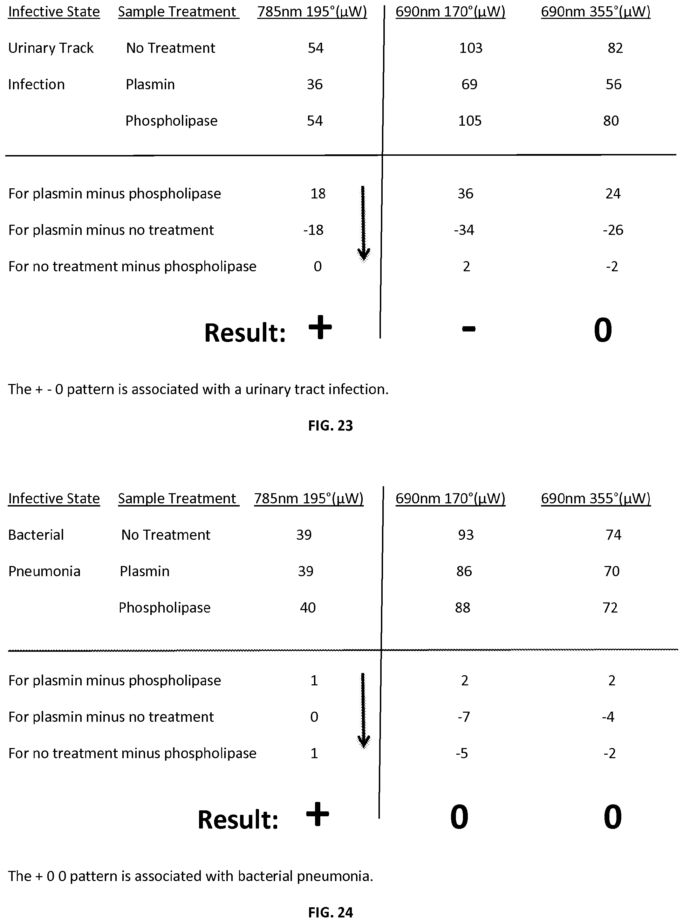

**Fig. 23** is a diagram showing light transmittance data at three different settings for three samples of whole blood obtained from an exemplary patient with a urinary tract infection. The samples were processed as follows: no treatment, treated with plasmin, or treated with phospholipase A₂. The values reported in the diagram are in microWatts.
**Fig. 24** is a diagram showing light transmittance data at three different settings for three samples of whole blood obtained from an exemplary patient with bacterial pneumonia. The samples were processed as follows: no treatment, treated with plasmin, or treated with phospholipase A₂. The values reported in the diagram are in microWatts.

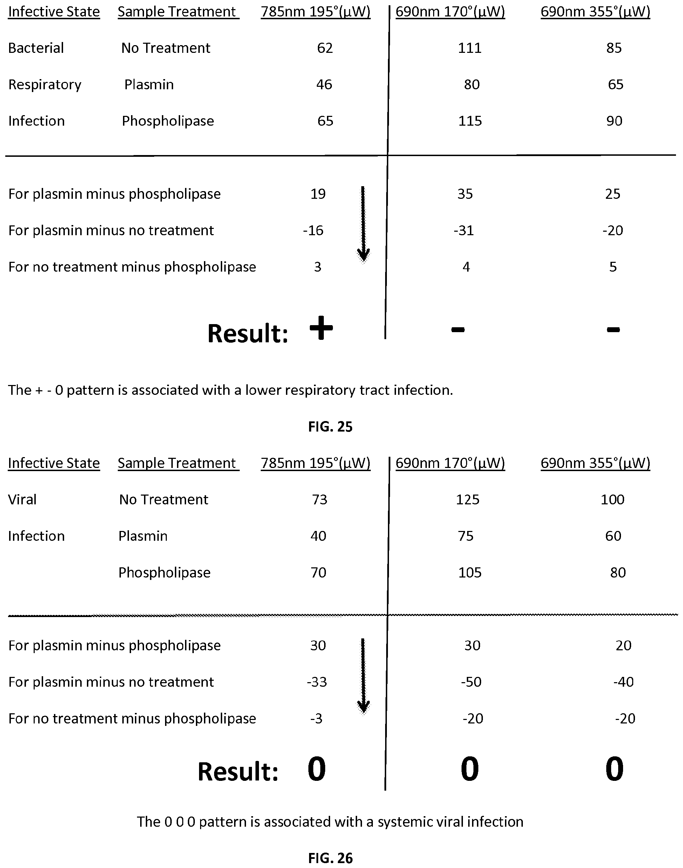

**Fig. 25** is a diagram showing light transmittance data at three different settings for three samples of whole blood obtained from an exemplary patient with a lower respiratory tract infection. The samples were processed as follows: no treatment, treated with plasmin, or treated with phospholipase A₂. The values reported in the diagram are in microWatts.
**Fig. 26** is a diagram showing light transmittance data at three different settings for three samples of whole blood obtained from an exemplary patient with a systemic viral infection. The samples were processed as follows: no treatment, treated with plasmin, or treated with phospholipase A₂. The values reported in the diagram are in microWatts.

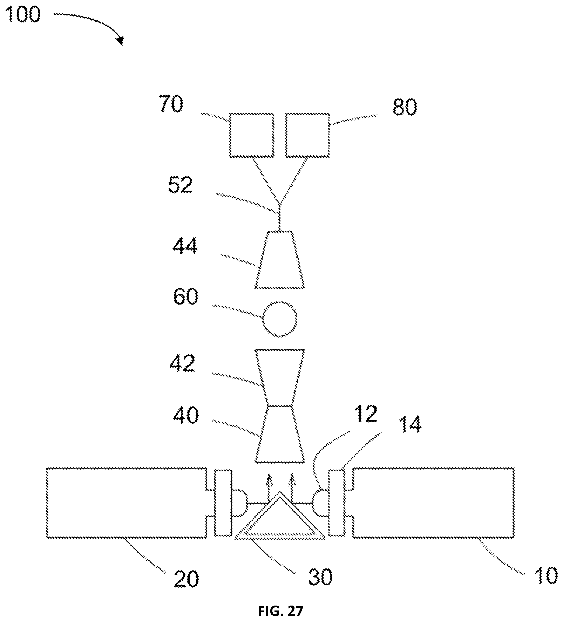

**Fig. 27** is a diagram of an second exemplary polarized optical system.

### DETAILED DESCRIPTION OF THE INVENTION

As described herein, it has been discovered that treatment of previously frozen and thawed blood samples with plasmin or phospholipase or with no enzyme changes the light transmittance properties of the blood samples such that a diagnostic analysis may be performed to identify a subject who is healthy or who has an infection, e.g., a bacterial or viral infection. This analysis is referred to herein as the Frozen Coagulation Analysis (FCA). Without wishing to be bound by theory, it is believed that the Frozen Coagulation Analysis (FCA) method is based on defining differences in the size, shape and composition of aggregates that are formed from fibrin and phospholipid membranes when the whole blood sample is frozen, thawed and then analyzed. In the absence of infection the vascular endothelium provides for regulation of blood fluidity and is also central to controlling coagulation by providing tissue factor, thrombin inhibitors and receptors for protein C activation. During infection disseminated intravascular coagulation (DIC) is initiated on the surface of endothelial cells that are located proximally to the general site of infection. The initiation if DIC results in the formation of fibrin and red blood cell complexes along the surface of the endothelium. Since there are variations in the specific architecture of various vasculature regions located within the bodies of humans and other mammals, there are differences in the exact physical and biochemical environment under which this coagulation event takes place. Red cell and fibrin complexes formed near the lungs experience a slightly different formation environment compared to, for instance, coagulation complexes formed near the abdomen or additionally in general circulation. The differences in formation environment result in different structural arrangements of red cell and fibrin complexes. Also without wishing to be bound by theory, it has been found herein that these differences result in a range of responses to enzymatic treatment, such as phospholipase or plasmin treatment ,and subsequent optical transmission and refraction properties. As described herein, it has been discovered that freezing and thawing of a blood sample produces aggregates of fibrin and phospholipid that take on specific configurations depending on the disease affecting the patient from whom the blood was drawn. Without wishing to be bound by theory, it is believed that the phospholipid fragments occluded by fibrin are processed by plasmin to free up the phospholipids. Addition of phospholipase produces an increase in availability of phospholipid fragments that result from incomplete digestion of the cell membrane that is bound up with fibrin. Additionally, it has been found that freezing whole blood has the added benefit of making the sample more optically transparent.

The freezing of whole blood prior to analysis teaches against the art. Bacterial and viral infections are classically known to cause some form of general coagulation dysfunction. Laboratory methodologies for coagulation testing universally stipulate that anticoagulant-treated whole blood samples should not be frozen prior to testing. Standard coagulation tests such as thromboelastometry, international normalized ratio, activated partial thromboplastin time, fibrinogen, antithrombin and thrombocytes require some level of preservation of the biochemical and rheologic conditions that were present when the sample was drawn. Freezing whole blood fractures phospholipid membranes releasing hemoglobin, intracellular components, and thromboplastic substances. Even moderate levels of blood cell lysis (0.9%) can influence the reliability of routine coagulation testing. While frozen plasma is acceptable for many coagulation tests, frozen whole blood is universally designated as not appropriate for coagulation testing.

Red cell membrane receptors interact with fibrinogen through an eptifibatide-sensitive receptor that in turn modifies the structure and lytic resistance of fibrin containing red blood cells RBCs (17). There are approximately 20 red blood cells (RBCs) for every platelet cell hence RBCs contribute a larger proportion of phospholipid mass to a blood sample compared to platelets. The action of freezing, thawing and then mixing causes the aggregation of fibrin and phospholipids.

Phospholipids become thromboplastic material by activating a cascade that begins with prothrombin and ultimately produces Fibrin Degradation Products (FDP's). Procoagulant phospholipids found on the surface of platelets are fibrin-adherent. The specific lipid environment at the platelet surface has the potential to influence fibrinogen binding to its receptor. In unfrozen platelets, outside-in signals generated by binding to a rigid fibrin network are essential for phosphatidylserine exposure after thrombin treatment (15). In a study of canine platelet preparations, frozen platelet preparations had increased amounts of phosphatidylserine in the outer leaflet of the platelet membrane and alterations in cellular morphology, all of which were consistent with platelet activation (16). Platelets play an important role in clot formation. The step of freezing platelets alters the natural state that was present when the sample was drawn. Freezing whole blood would negatively impact any prior art method to accurately measure sample platelet function or count.

As described herein, whole blood samples were obtained from subjects having various types of infections (e.g., bacterial, fungal or viral infections) or having no infection. The blood samples were treated with anticoagulant, subsequently frozen and thawed and then either processed with various enzymes (e.g., plasmin or phospholipase A₂) or not processed with such enzymes. It was unexpectedly discovered that the transmittance of polarized light over multiple wavelengths through the various differently-processed blood samples after a freeze-thaw cycle identified not only the presence or absence of infection in a subject, but also the type of infection (e.g., blood stream infection, urinary tract infection, bacterial pneumonia, lower respiratory tract infection, or systemic viral infection). Further, it was surprisingly found that the use of polarized light caused flowing and rotation of the aggregates within the blood samples, such that many aggregates passed in front of the optical sensors, improving the quality of the light sampling and amplifying the differences between the different types of samples (infection versus no infection, as well as the type of infection). Thus, methods and kits for analyzing processed blood samples as described above are useful, e.g., to identify subjects having or not having infections or to triage subjects for further identification of infections and/or treatment. Identification of the presence/absence and type of infections is helpful in ensuring that subjects with infections receive proper treatment and/or further testing and that subjects without infection are not treated unnecessarily.

### Methods

Aspects of the disclosure relate to methods of assaying a (i.e., at least one) blood sample obtained from a subject. In some embodiments, the method comprises providing one or more samples of anticoagulant-treated blood from the subject, contacting at least one of the samples with an enzyme as described herein (e.g., plasmin and/or phospholipase A₂) and measuring transmittance of artificial light (e.g., polarized light) passed through the one or more samples after the contacting.

In some embodiments, the method comprises (a) providing a first and a second anticoagulant-treated blood sample that have been previously frozen and thawed; (b) contacting the second anticoagulant-treated blood sample, but not the first sample, with: (i) plasmin as described herein or plasminogen and a plasminogen activator as described herein; or (ii) an enzyme that will break down a phospholipid bilayer as described herein; (c) passing light from an artificial source through the first and second anticoagulant-treated blood samples; and (d) measuring transmittance of the light through the first and second anticoagulant-treated blood samples.

In some embodiments, the method comprises (a) providing a first, a second, and a third anticoagulant-treated blood sample that have been previously frozen and thawed; (b) contacting the second anticoagulant-treated blood sample but not the first, with plasmin or plasminogen and a plasminogen activator and contacting the third anticoagulant-treated blood sample, but not the first, with an enzyme that will break down a phospholipid bilayer; c) passing light from an artificial source through the first, second and third anticoagulant-treated blood samples; and d) measuring transmittance of the light through the first, second, and third anticoagulant-treated blood samples.

In some embodiments, the method comprises (a) providing an anticoagulant-treated blood sample that have been previously frozen and thawed; (b) contacting the anticoagulant-treated blood sample with an enzyme that will break down a phospholipid bilayer; (c) passing light from an artificial source through the anticoagulant-treated blood sample; and (d) measuring transmittance of the light through the anticoagulant-treated blood sample.

In some embodiments of any one of the methods provided herein, the method further comprises freezing and/or thawing the one or more anticoagulant-treated blood samples (e.g., a first, second, and/or third anticoagulant-treated blood sample described herein).

In some embodiments of any one of the methods provided herein, the method further comprises comparing the transmittance of the light through the first anticoagulant-treated blood sample with the transmittance of light through the second and/or third anticoagulant-treated blood samples. In some embodiments, the method further comprises comparing the transmittance of the light through the second anticoagulant-treated blood sample with the transmittance of light through the third anticoagulant-treated blood sample. In some embodiments, the comparing comprises subtracting one or more transmittance values from one or more other transmittance values. In some embodiments, the method comprises subtracting the transmittance of light through the second anticoagulant-treated blood sample from the transmittance of light through the first anticoagulant-treated blood sample (or vice versa) and subtracting the transmittance of light through the third anticoagulant-treated blood sample from the transmittance of light through the first anticoagulant-treated blood sample (or vice versa). In some embodiments, the method further comprises subtracting the transmittance of light through the third anticoagulant-treated blood sample from the transmittance of light through the second anticoagulant-treated blood sample. In some embodiments, the method further comprises subtracting the transmittance of light through the second anticoagulant-treated blood sample from the transmittance of light through the third anticoagulant-treated blood sample.

In some embodiments, where the light is at a first and a second wavelength, the comparison may comprise comparing the transmittance of the light at the first wavelength and the second wavelength through the first anticoagulant-treated blood sample with the transmittance of light at the first wavelength and the second wavelength through the second and/or third anticoagulant-treated blood samples. In some embodiments, the method further comprises comparing the transmittance of light at the first wavelength and the second wavelength through the second anticoagulant-treated blood sample with the transmittance of light at the first wavelength and the second wavelength through the third anticoagulant-treated blood sample. In some embodiments, the method further comprises subtracting the transmittance of light at each wavelength through the second anticoagulant-treated blood sample from the transmittance of light at each wavelength through the first anticoagulant-treated blood sample (or vice versa) and subtracting the transmittance of light at each wavelength through the third anticoagulant-treated blood sample from the transmittance of light at each wavelength through the first anticoagulant-treated blood sample (or vice versa). For example, the transmittance of light at the first wavelength through the second sample is subtracted from the first wavelength through the first sample, the second wavelength through the second sample is subtracted from the second wavelength through the first sample, the first wavelength through the third sample is subtracted from the first wavelength through the first sample, and the second wavelength through the third sample is subtracted from the second wavelength through the first sample, etc. In some embodiments, if multiple polarizer angles are utilized for each wavelength, then the transmittance of light for each polarizer angle may also be used in the comparison or subtraction. Comparisons and/or subtractions may be accomplished using any method known in the art and may be aided by use of a calculator or computer. In some embodiments, the comparing or subtracting yields one or more values. In some embodiments of any one of the methods described herein, the method further comprises correlating a value obtained by the comparing or subtracting to a condition (e.g., infection or no infection). In some embodiments, a number of values obtained by the comparing or subtracting may be correlated to a condition (e.g., infection or no infection). In some embodiments, the values obtained are expressed as either a positive number, a negative number or zero. In embodiments described below, various combinations of positive and negative numbers (and optionally zeroes) are characteristic of particular conditions.

In some embodiments of any one of the methods provided herein, the method further comprises converting the measured transmittance(s) or comparisons or subtractions into a diagnostic indicator, e.g., converting the measured transmittance(s) or comparisons or subtractions into an indication of presence, absence, and/or type of infection, e.g., as a diagram, word(s), or symbol(s).

In some embodiments of any one of the methods provided herein, the method further comprises identifying a subject as having or not having an infection based on the measured transmittance(s) or comparisons or subtractions described herein.

In some embodiments of any one of the methods provided herein, the method further comprises recording the measured transmittance(s), comparisons, subtractions, diagnostic indicator, and/or identification of the subject, such as in a database or chart.

In some embodiments of any one of the methods provided herein, a subject identified as having an infection or not having an infection may be subjected to further testing or recommended to receive further testing, e.g., a further diagnostic test known in the art for bacterial, viral, or fungal infection. In some embodiments, further testing comprises one or more nucleic acid-based assays, e.g., PCR assays. Such nucleic acid-based assays may be used, e.g., to identify a genus, family, and/or species of bacteria, virus, or fungus.

In some embodiments of any one of the methods provided herein, a subject identified as having an infection may be hospitalized or recommended to go to a hospital. In some embodiments of any one of the methods provided herein, a subject identified as not having an infection may be recommended to not go to a hospital. In some embodiments of any one of the methods provided herein, a subject identified as having an infection may be treated with a therapeutic agent, e.g., such as an antibacterial, antifungal or antiviral therapeutic agent. In some embodiments of any one of the methods provided herein, a subject identified as having an infection may be recommended a treatment with a therapeutic agent, e.g., such as an antibacterial, antifungal or antiviral therapeutic agent. Such therapeutic agents are known in the art. Non-limiting exemplary antibacterial therapeutic agents include penicillins, cephalosporins, polymyxins, rifamycins, lipiarmycins, quinolones, sulfonamides, macrolides, lincosamides, tetracyclines, aminoglycosides, cyclic lipopeptides, glycylcyclines, oxazolidinones, and lipiarmycins. Non-limiting exemplary antifungal therapeutic agents include polyenes, imidazoles, triazoles, thiazoles, allylamines, echinocandins, benzoic acid, ciclopirox olamine, flucytosine, griseofulvin, haloprogin, tolnaftate, undecylenic acid, and crystal violet. Non-limiting exemplary antiviral therapeutic agents include zanamivir, oseltamivir, amantadine and rimantadine.

Other aspects of the disclosure relate to a method of selecting a subject for therapy. In some embodiments, the method comprises selecting a subject for therapy on the basis of one or more measured transmittances, the results of comparing the one or more measured transmittances, or the results of subtracting the one or more measured transmittances for each other as described herein. For example, a subject identified as having an infection as described herein may be selected for treatment with a therapy, such as an antibacterial, antifungal or antiviral therapeutic agent known in the art or described herein.

### Light and Measuring Transmittance of Light

Aspects of the disclosure relate to passing light from an artificial source through one or more anti-coagulant treated blood sample(s) described herein (e.g., that has been previously frozen and thawed and optionally treated with one or more enzymes described herein) and (d) measuring transmittance of the light through the anti-coagulant treated blood sample(s). In a preferred embodiment, the light is polarized light. In some embodiments, measuring transmittance of light includes measuring refraction of polarized light, e.g., using a bifurcated optical cable system to detect the transmittance of the refracted light.

In some embodiments of any one of the methods provided herein, the light (e.g., polarized light) is at a wavelength of between 750-820 nm (e.g., 750, 755, 760, 765, 770, 775, 780, 785, 790, 795, 800, 805, 810, 815, or 820 nm, or any integer in between). In some embodiments of any one of the methods provided herein, the light (e.g., polarized light) is at a wavelength of between 775-795 nm (e.g., 775, 780, 785, 790, or 795 nm, or any integer in between). In some embodiments of any one of the methods provided herein, the light (e.g., polarized light) is at a wavelength of 785 nm. In some embodiments of any one of the methods provided herein, the light (e.g., polarized light) is at a wavelength of between 650-730 nm (e.g., 650, 655, 660, 665, 670, 675, 680, 685, 690, 695, 700, 705, 710, 715, 720, 725, or 730 nm, or any integer in between). In some embodiments of any one of the methods provided herein, the light (e.g., polarized light) is at a wavelength of between 680-700 nm (e.g., 680, 685, 690, 695 or 700 nm, or any integer in between). In some embodiments of any one of the methods provided herein, the light (e.g., polarized light) is at a wavelength of 690 nm. In some embodiments of any one of the methods provided herein, the light (e.g., polarized light) is at a first and a second wavelength, which are different. The first and second wavelength may be measured at the same time or at different times (e.g., sequentially, as in the first wavelength is measured at a first time, then the second wavelength is measured at a second time or vice versa). In some embodiments, the first wavelength is between 750-820 nm (e.g., 750, 755, 760, 765, 770, 775, 780, 785, 790, 795, 800, 805, 810, 815, or 820 nm, or any integer in between) and the second wavelength is between 650-730 nm (e.g., 650, 655, 660, 665, 670, 675, 680, 685, 690, 695, 700, 705, 710, 715, 720, 725, or 730 nm, or any integer in between). In some embodiments, the first wavelength is between 775-795 nm (e.g., 775, 780, 785, 790, or 795 nm, or any integer in between) and the second wavelength is between 680-700 nm (e.g., 680, 685, 690, 695 or 700 nm, or any integer in between). In some embodiments, the first wavelength is 785 nm and the second wavelength is 690 nm.

The artificial source of light may be any artificial source of light known in the art or described herein capable of passing light through a sample, e.g., passing polarized light through a sample. In some embodiments, the artificial source of light is one or more lasers, e.g., a 785 nm laser and a 690 nm laser. In some embodiments, the artificial source of light is a lamp. Lasers, lamps, and other such artificial sources of light are known in the art and commercially available (see, e.g., products from Lasermate Group, Inc., Walnut, California). In some embodiments, the light from the artificial source is polarized or further polarized prior to being passed through the sample using one or more polarizers, reflective surfaces (e.g., mirrors), collimators, or combinations thereof. In some embodiments, the light from the artificial source, e.g., a lamp, is filtered prior to being passed through the sample to achieve a specific wavelength described herein.

The transmittance of light (e.g., of polarized light) may be measured using any method known in the art or described herein, e.g., using a photodiode power sensor or a charge-coupled device (CCD) camera. In some embodiments, the transmittance is measured using a photodiode power sensor. In some embodiments, the light is transmitted through one or more collimators and/or filters prior to the measurement. In some embodiments, the light is transmitted through a bifurcated optical cable system, e.g., wherein each cable transmits a light signal to a separate photodiode power sensor. In some embodiments, where two photodiode power sensors are employed, each power sensor may be equipped with a filter (e.g., one equipped with a filter for 690 nm wavelength light and the other equipped with a filter for 785 nm wavelength light).

In some embodiments, polarized light is passed through each anticoagulant-treated blood sample (e.g., that has been previously frozen and thawed and optionally treated with one or more enzymes described herein). In some embodiments, the polarized light is at a wavelength of between 750-820 nm (e.g., between 775-795 nm, such as 785 nm). In some embodiments, the polarized light is at a wavelength of between 650-730 nm (e.g., between 680-700 nm, such as 690 nm). In some embodiments, polarized light is passed through each anticoagulant-treated blood sample at a first and second wavelength. In some embodiments, the first wavelength is between 750-820 nm (e.g., between 775-795 nm). In some embodiments, the second wavelength is between 650-730 nm (e.g., between 680-700 nm). In some embodiments, light from an artificial light source, e.g., a laser, is passed through a polarizer, where the polarizer is set at one or more angles, where, if the polarizer is mounted perpendicular to the earth (optionally parallel to a mirror), a line bisecting the 0° and 180° coordinates of the polarizer will be perpendicular to the earth. In some embodiments, if the polarized light is passed through each anticoagulant-treated blood sample at a first and second wavelength. The first and second wavelength may be polarized using a polarizer set at one or more angles.

In some embodiments, the polarized light is at a wavelength of between 750-820 nm (e.g., between 775-795 nm) and the angle of the polarizer is between 130-200°. In some embodiments, the angle is between 130-150°. In some embodiments, the angle is 140°. In some embodiments, the angle is between 180-200°. In some embodiments, the angle is 195°. In some embodiments, the angle is two angles.

In some embodiments, the polarized light is at a wavelength of 650-730 nm (e.g., between 680-700 nm, such as 690 nm) and the angle of the polarizer is between 70-100°, between 160-200° or between 340-370°. In some embodiments, and the angle of the polarizer is between 70-100°. In some embodiments, the angle is two angles. In some embodiments, the two angles are each independently between 70-100°. In some embodiments, one angle is 80° and the other angle is 95°. In some embodiments, one angle is between 160-200° and the other angle is between 340-370°. In some embodiments, one angle is 170° and the other angle is 355°.

In some embodiments, light at the following wavelengths is passed through polarizers at the following angles and then passed through each anticoagulant-treated blood sample (which may each be measured separately, e.g., using one or more photodiode power sensors):
(a) 775-795 nm (e.g., 785 nm) at a polarizer angle of 195°;
(b) 680-700 nm (e.g., 690 nm) at a polarizer angle of 355°; and
(c) 680-700 nm (e.g., 690 nm) at a polarizer angle of 170°.

In some embodiments, light at the following wavelengths is passed through polarizers at the following angles and then passed through each anticoagulant-treated blood sample (which may each be measured separately, e.g., using one or more photodiode power sensors):
(a) 775-795 nm (e.g., 785 nm) at a polarizer angle of 140°;
(b) 680-700 nm (e.g., 690 nm) at a polarizer angle of 80°; and
(c) 680-700 nm (e.g., 690 nm) at a polarizer angle of 95°.

### Enzymes

Aspects of the disclosure relate to one or more enzymes and their use in kits and methods described herein.

In some embodiments, the one or more enzymes include plasmin or, alternatively, plasminogen and a plasminogen activator. The plasminogen and plasminogen activator may be contacted directly with an anticoagulant-treated blood sample described herein, or may be combined first to produce plasmin. The produced plasmin may then be contacted with an anticoagulant-treated blood sample described herein. In some embodiments, a urokinase may be used in combination with the plasminogen and the plasminogen activator (e.g., streptokinase or a tissue plasminogen activator). Any one or more of the enzymes described herein may be produced recombinantly or isolated/extracted and/or purified from biological samples (e.g., tissue or fluid samples) or by any other method known in the art.

The plasmin may be, e.g., mammalian plasmin, such as human, porcine or bovine plasmin. Plasmin may be obtained using any method known in the art or from a commercial vendor. For example, plasmin may be extracted and/or purified from plasma (e.g. human, porcine or bovine plasma) or may be recombinantly produced (e.g., in CHO cells) and is also commercially available (see. e.g., Sigma-Aldrich, Catalog Number: P1867). Plasmin may also be produced, e.g., by combining plasminogen with a plasminogen activator. For example, plasminogen may be contacted with streptokinase and urokinase to produce plasmin. Plasminogen and plasminogen activators are described herein. Plasminogen from any source (e.g., bovine, rabbit, or horse) may be converted to plasmin with any plasminogen activator from any source.

The plasminogen may be, e.g., mammalian plasminogen, such as human, rabbit, horse, porcine or bovine plasminogen. Plasminogen may be obtained using any method known in the art or from a commercial vendor. For example, plasminogen may be extracted and/or purified from plasma (e.g., human, rabbit, horse, porcine or bovine plasma) or may be recombinantly produced (e.g., in CHO cells) and is also commercially available (see. e.g., Sigma-Aldrich, Catalog Numbers: P9156, P5661, and P7397, and CAS Number: 9001-91-6).

The plasminogen activator may be, e.g., a mammalian tissue plasminogen activator, such as human, porcine or bovine tissue plasminogen activator. In some embodiments, the plasminogen activator is streptokinase, urokinase, tissue plasminogen activator, or combinations thereof. Plasminogen activator may be obtained using any method known in the art or from a commercial vendor. For example, plasminogen activator may be extracted and/or purified from tissue or cells (e.g. human, porcine, or bovine tissue or cells, or from streptococcus cells) or may be recombinantly produced (e.g., in CHO cells or *E. coli* cells) and is also commercially available (see. e.g., Sigma-Aldrich Catalog Numbers: T4825, T5451, T0831, S3134, S0827, S0577, and CAS Number: 9002-01-1). The urokinase may be, e.g., a mammalian urokinase, such as human, porcine or bovine urokinase. Urokinase may be extracted and/or purified from urine or other tissue (e.g., human, porcine, or bovine urine or kidney tissue) and is also commercially available (see. e.g., Sigma-Aldrich, Catalog Number: U0633 and U4010, CAS Number: 9039-53-6).

In some embodiments, the one or more enzymes include an enzyme that will break down a phospholipid bilayer. Examples of enzymes that will break down a phospholipid bilayer include, but are not limited to, phospholipase A. In some embodiments, the enzyme that will break down a phospholipid bilayer is phospholipase A₂. Phospholipase A₂ can be extracted and/or purified from venom, e.g., honey bee venom or snake venom, or mammalian tissue, e.g., porcine or bovine pancreas, or may be recombinantly produced (e.g., in CHO cells or *E. coli* cells) and is also commercially available (see, e.g., Sigma-Aldrich, Catalog Numbers: P9279, P8913, P6534, P5910, CAS Number: 9001-84-7). The phospholipase A₂ can also be lyophilized and/or powdered.

In some embodiments of any one of the methods described herein, an anticoagulant-treated blood sample is contacted with 0.1-10U (e.g., 0.5-5U) plasminogen. In some embodiments of any one of the kits described herein, the kit comprises 0.1-10U (e.g., 0.5-5U) plasminogen. In some embodiments of any one of the methods described herein, an anticoagulant-treated blood sample is contacted with 50-50,000U (e.g., 500-5,000U) streptokinase. In some embodiments of any one of the kits described herein, the kit comprises 50-50,000U (e.g., 500-5,000U) streptokinase. In some embodiments of any one of the methods described herein, an anticoagulant-treated blood sample is contacted with 10-40,000 U (e.g., 100-4,000 U) Phospholipase A₂. In some embodiments of any one of the kits described herein, the kit comprises 10-40,000 U (e.g., 100-4,000 U) Phospholipase A₂. In some embodiments of any one of the methods described herein, an anticoagulant-treated blood sample is contacted with 0.001- 100 U (e.g., 0.01- 10 U) urokinase. In some embodiments of any one of the kits described herein, the kit comprises 0.001- 100 U (e.g., 0.01-10 U) urokinase. In some embodiments, plasmin is produced and used in a method or contained within a kit, wherein the plasmin is produced by combining 0.1-10U (e.g., 0.5-5U) plasminogen, 50-50,000U (e.g., 500-5,000U) streptokinase, and 0.001- 100 U (e.g., 0.01- 10 U) urokinase.

In some embodiments of any one of the methods described herein, an anticoagulant-treated blood sample is contacted with 0.1-10U (e.g., 0.5-5U) plasminogen, 50-50,000U (e.g., 500-5,000U) streptokinase and 0.001- 100 U (e.g., 0.01- 10 U) urokinase. In some embodiments of any one of the methods described herein, an anticoagulant-treated blood sample is contacted with plasmin produced by combining .1-10U (e.g., 0.5-5U) plasminogen, 50-50,000U (e.g., 500-5,000U) streptokinase and 0.001- 100 U (e.g., 0.01- 10 U) urokinase. In some embodiments of any one of the methods described herein, an anticoagulant-treated blood sample is contacted with 10-40,000 U (e.g., 100-4,000 U) Phospholipase A₂. In some embodiments of any one of the kits described herein, the kit comprises .1-10U (e.g., 0.5-5U) plasminogen, 50-50,000U (e.g., 500-5,000U) streptokinase, 0.001- 100 U (e.g., 0.01- 10 U) urokinase, and 10-40,000 U (e.g.,100-4,000 U) Phospholipase A₂.

In some embodiments of any one of the methods described herein, an anticoagulant-treated blood sample is contacted with one or more of the enzymes described herein and incubated before passing light through the sample. In some embodiments, the sample is incubated for at least 30 seconds or at least 1, at least 2, at least 3, at least 4, at least 5, at least 6, at least 7, at least 8, at least 9 or at least 10 minutes before passing light through the sample. In some embodiments, the sample is incubated for 30 seconds to 1 hour, 30 seconds to 30 minutes, 30 seconds to 10 minutes or 30 seconds to 5 minutes.

In some embodiments of any one of the methods described herein, an anticoagulant-treated blood sample is contacted with water or a buffered solution, which may further comprise one or more enzymes described herein.

### Samples

Aspects of the disclosure relate to anticoagulant-treated blood sample(s) and their use in any one of the methods or kits described herein. The anticoagulant may be any anticoagulant known in the art, e.g., any one or more of ethylenediaminetetraacetic acid (EDTA), sodium polyanetholesulfonate (SPS), coumarin, heparin, batroxobin, hementin, citrate, oxalate, dabigatran, rivaroxaban and apixaban. In some embodiments, the anticoagulant is EDTA.

In some embodiments, the sample may be, or may be derived from, whole blood isolated from a subject who may have or is suspected of having an infection, e.g., a bacterial, viral or fungal infection. Subjects are further described herein. In some embodiments, a single whole blood draw from a subject may be split into multiple samples, e.g., multiple anticoagulant-treated blood sample(s). In some embodiments, multiple whole blood draws from a subject may be used to produce multiple samples, e.g., multiple anticoagulant-treated blood sample(s). Whole blood may be isolated using any method known in the art, e.g., by venipuncture (see, e.g., WHO guidelines on drawing blood: best practices in phlebotomy, ISBN 978 82 4 159922 1, World Health Organization, Switzerland). The whole blood may be contacted with the anticoagulant, such as by adding anticoagulant to the whole blood or by collecting the whole blood into a container that contains anticoagulant (e.g., in a blood collection tube such as a Vacutainer®, which contains an anticoagulant).

In some embodiments, the anticoagulant-treated blood sample(s) may be diluted and/or further processed, such as by adding water or a buffered solution. In some embodiments, the anticoagulant-treated blood sample(s) are previously frozen and thawed prior to their use in any one of the methods or kits described herein. In some embodiments, any one of the methods or kits described herein comprises thawing previously frozen anticoagulant-treated blood sample(s) prior to contacting the blood sample(s) with one or more enzymes described herein. In some embodiments, any one of the methods described herein comprises freezing and/or thawing the anticoagulant-treated blood sample(s) prior to contacting the blood sample(s) with one or more enzymes described herein. The anticoagulant-treated blood sample(s) may be frozen and thawed using any method known in the art (e.g., freezing by storage in a freezer or by contacting with dry ice or liquid nitrogen and thawing by incubation at room temperature (e.g., 23-25 degrees Celsius) or in a water bath (e.g., at 23-37 degrees Celsius)). In some embodiments, the anticoagulant-treated blood sample(s) are frozen for at least 30 seconds or at least 1, at least 2, at least 3, at least 4, at least 5, at least 6, at least 7, at least 8, at least 9, at least 10, or at least 30 minutes or at least 1, at least 2, at least 3, at least 4, at least 5, at least 6, at least 7, at least 8, at least 9, or at least 10 hours prior to thawing. In some embodiments, the anticoagulant-treated blood sample(s) are frozen for 30 seconds to 48 hours, 30 seconds to 24 hours, 30 seconds to 8 hours, 30 seconds to 1 hours, 30 seconds to 30 minutes, 30 seconds to 10 minutes or 30 seconds to 5 minutes.

In some embodiments, multiple anticoagulant-treated blood sample(s) are contemplated (e.g., a first, second, third, fourth, fifth, etc. anticoagulant-treated blood sample(s)). The use of "first", "second", "third", etc. as used herein does not imply a particular order of importance or timing unless specifically stated otherwise. In some embodiments, a first and a second anticoagulant-treated blood sample are contemplated for use in any one of the methods or kits described herein. In some embodiments, a first, a second, and a third anticoagulant-treated blood sample are contemplated for use in any one of the methods or kits described herein. In some embodiments, any one or more of the anticoagulant-treated blood sample(s) are contacted with one or more enzymes described herein (e.g., contacted with (i) plasmin or plasminogen and a plasminogen activator; or (ii) an enzyme that will break down a phospholipid bilayer).

In some embodiments, the anticoagulant-treated blood sample(s) of any one of the methods or kits described herein is a liquid and light as described herein is passed through the liquid. In some embodiments, the anticoagulant-treated blood sample(s) of any one of the methods or kits described herein is not agitated prior to use in the method or kit, e.g., is not agitated such that a foam is produced.

### Subjects

Aspects of the disclosure relate to subjects, such as subjects having or suspected of having an infection, such as a bacterial, viral, or fungal infection. In some embodiments, the subject has or is suspected of having a blood stream infection, a urinary tract infection, bacterial pneumonia, a lower respiratory tract infection, an infection in the bile and urine, a skin infection, or systemic viral infection. In some embodiments, the subject has one or more symptoms associated with infection. Symptoms of infection are known in the art and identifiable to the skilled practitioner. Examples of symptoms of infection include, but are not limited to, fever, cough, sore throat, runny nose, headache, fatigue, muscle aches, redness, peeling, cracking or blistering of the skin, itching sensations, burning sensations, gastrointestinal pain, diarrhea, nausea, vomiting, a respiratory rate greater than 20 breaths per minute, heart rate higher than 90 beats per minute, and/or abrupt change in mental status or abdominal pain.

Examples of bacteria that may cause infection in a subject and are suitable for the kits and methods described herein include, but are not limited to, bacteria of the genus *Streptococcus, Salmonella, Bacillus, Clostridium, Pseudomonas, Neisseria, Mycobacterium, Yersinia, Haemophilus, Bordetella, Listeria, Treponema, Chlamydia, Brucella,* or *Vibrio.* Examples of viruses that may cause infection in a subject and are suitable for the kits and methods described herein include, but are not limited to, viruses of the genus *Lymphocryptovirus, Simplexvirus, Roseolovirus, Lentivirus, Mupapillomavirus, Alphapapillomavirus, Respirovirus, Enterovirus, Betacoronavirus, Influenzavirus A, Influenzavirus B, Influenzavirus C, Morbilivirus, Rubulavirus, Lyssavirus, Rotavirus, Rubivirus,* and *Flavivirus.* Examples of fungi that may cause infection of a subject and are suitable for the kits and methods described herein include, but are not limited to, fungi of the genus *Histoplasma* (e.g., *Histoplasma capoulatum), Coccidioides* (e.g., *C. immitis), Blastomyces* (e.g., *B. dermatitidis), Candida, Aspergillus, Cryptococcus,* and *Zygomycetes.*

### Kits

Yet other aspects of the disclosure relate to kits. In some embodiments, the kit comprises phospholipase A (e.g., phospholipase A₂) as described herein, plasmin or plasminogen and plasminogen activator as described herein, and a sample cartridge or chamber for optical analysis. The sample cartridge or container may be e.g., a cuvette, a slide, or a cartridge described herein (e.g., FIGs. 14 and 15). In some embodiments of any one of the kits described herein, the kit comprises 1-10U (e.g., 0.5-5U) plasminogen, 50-50,000U (e.g., 500-5,000U) streptokinase, 0.001- 100 U (e.g., 0.01- 10 U) urokinase, and 10-40,000 U (e.g., 100-4,000 U) Phospholipase A₂. In some embodiments of any one of the kits described herein, the kit comprises plasmin produced by combining 1-10U (e.g., 0.5-5U) plasminogen, 50-50,000U (e.g., 500-5,000U) streptokinase, and 0.001- 100 U (e.g., 0.01- 10 U) urokinase.

### Devices

Other aspects of the disclosure relate to a device for measuring transmittance of light (e.g., polarized light) through one or more anticoagulant-treated blood samples. In some embodiments, the device comprises the device shown in FIG. 27. In some embodiments, the device comprises the device shown in **FIG. 17****.** In some embodiments, one or more anticoagulant-treated blood sample(s) are placed into one or more sample cartridge(s) (e.g., FIGs. 14 and 15) and the sample cartridge(s) is/are loaded into the device (e.g., FIG .17). Any device described herein may further comprise one or more stand(s) and/or base(s) to stabilize the optical system.

### Other Embodiments

Other non-limiting embodiments of the disclosure are described below. In some embodiments, 3 X 300 µl whole EDTA blood was frozen to a solid at - 20° C, thawed at room temperature and distributed into three separate 8mm x 40mm glass Shell Vials. For the first vial 400 µl water was added (the no treatment sample). To the second vial was added 0.5 - 5U bovine plasminogen, 500 - 5,000 U streptokinase and 0.01 - 10 U human urokinase (Sigma Aldrich) in 400 µl water. To the third vial was added 100 - 4,000 U Phospholipase A2 (Sigma Aldrich) in 400 µl water. Each vial was gently mixed and incubated at room temperature for 2 minutes then exposed to the exemplary FCA Polarized Optical System described below and shown in FIG. 27.

In some embodiments, the exemplary FCA Polarized Optical System was composed of appositionally placed lasers **10, 20** (e.g., Lasermate 785 nm Cat. # IML 785-800FLD and Lasermate 690 nm Cat. # RML690-800FLD lasers) each combined with polarizers **12** (e.g., Thor Labs nanoparticle linear polarizers Cat. # LPVIS050-MP) mounted on rotating mounts **14** (e.g., Thor Labs motorized precision rotation stages with servo drivers, Cat. # PRM1Z8E). The rotating mount was positioned so that a line transecting the 0° and 190° positions was parallel to the earth. A mirror **30** (e.g., Thor Labs knife edge right angle prism dielectric mirror, Cat. # MRAK25-E02 and Collimation Package Cat. # F810SMA-780) may be used to direct the laser output(s) into a series of collimators **40, 42** (e.g., Ocean Optics 84-series Collimating Lens) serving to shape the beam and appropriately illuminate the sample.

In some embodiments, after passing through the collimators **40, 42,** the light is transmitted through the sample cuvette **60** and into another collimator **44** (e.g., Ocean Optics 84-series Collimating Lens), for further processing of the transmitted light. The collimator **44** may be attached to a bifurcated optical fiber assembly **52** (e.g., Thor Labs Bifurcated Optical Fiber Assembly Cat. # Cat. # QBIF600-VIS-NIR) where light signal is supplied to separate photodiode power sensors (e.g., integrating sphere sensors from Thor Labs Cat. # S142C). Each power sensor may be equipped with appropriate filters **70, 80** (e.g., Semrock Brightline filter FF01-689/23-12.5 for 690 nm wavelength light and Semrock MaxLine filter number LL01-785-12.5 for 785 nm wavelength light, respectively).

In some embodiments, the polarizer for the 785 nm laser was set at 195°. In some embodiments, the polarizer for the 690 nm laser was set at 355°. In some embodiments, ten seconds after sample introduction to the light beam, readings from a Thorlabs' PM200 Touch Screen Power and Energy Meter were recorded for the 785 nm and the 690 nm signals. In some embodiments, the polarizer for the 690 nm laser was then rotated to the 170° setting and the signal was recorded after 10 seconds. In some embodiments, these readings are processed in order to derive a three position diagnostic code made up of +'s, -'s and 0's.

In figures 21 - 26 are listed readings for various exemplary samples taken from various clinical cases. In the upper half of each figure are listed the actual energy readings in micro watts (µW). Below these data blocks are numbers that represent the differences between 1) plasmin and phospholipase, 2) no treatment and plasmin and 3) no treatment and phospholipase. For the 785 nm reading, the 195° designation describes the setting used to position the polarizer for that laser. For the 690 nm laser, readings were taken when the polarizer was positioned at either 170° or 355°.

In some embodiments, for the 785 nm laser, the differences are listed and the direction that would yield a valid calculation is noted. In some embodiments, if the calculation proceeds from bottom to top, the patient from whom the blood was drawn is designated as not having a viral or bacterial infection (Fig. 21). In some embodiments, if the calculation for the differences proceeds downward the patient is designated as having a viral or bacterial infection (Fig. 22 - 26). In this case of an infection positive determination a "+" is recorded. In some embodiments, if two of the calculated differences (that proceed downward) result in negative numbers a "0" is recorded.

In some embodiments, for the 690 nm laser the differences between 1) plasmin and phospholipase, 2) no treatment and plasmin and 3) no treatment and phospholipase are derived as with the 785 nm readings but designating a number as negative or positive is based on the following rule. In some embodiments, when calculating the difference between plasmin and phospholipase treated samples a number is designated as negative if the phospholipase reading is lower than the plasmin reading. In some embodiments, a zero difference is of course left as zero. In some embodiments, when calculating the difference between the no treatment and plasmin treated samples the resulting number is designated as negative if the plasmin reading is lower than the no treatment reading. In some embodiments, when calculating the difference between the no treatment and phospholipase treated samples the resulting number is designated as negative if the phospholipase reading is lower than the no treatment reading.

In some embodiments, for the 690 nm readings at the 170° or 355° orientations of the polarizers, when the plasmin minus phospholipase number is negative a "-" designation is made in the second position of the diagnostic code. In some embodiments, when two of the numbers are negative a "0" designation is made for the diagnostic code. In some embodiments, if all three of the numbers are negative a "-" designation is made.

In some embodiments, the - - - pattern is associated with a blood culture negative condition. In some embodiments, the + + + pattern is associate with a blood stream infection. In some embodiments, the + - 0 pattern is associated with a urinary tract infection. In some embodiments, the + 0 0 pattern is associated with bacterial pneumonia. In some embodiments, the + - - pattern is associated with a lower respiratory tract bacterial infection. In some embodiments, the 0 0 0 pattern is associated with a systemic viral infection.

Without further elaboration, it is believed that one skilled in the art can, based on the above description, utilize the present disclosure to its fullest extent. The following specific embodiments are, therefore, to be construed as merely illustrative, and not limitative of the remainder of the disclosure in any way whatsoever. All publications cited herein are incorporated by reference for the purposes or subject matter referenced herein.

### EXAMPLES

### Example 1: FCA assay

Whole blood samples were taken from various confirmed clinical cases of various infection types and the anticoagulant EDTA was added. Each clinical case presented with one of the following: no infection, a blood stream infection, a urinary tract infection, bacterial pneumonia, lower respiratory tract infection, or systemic viral infection. 3 X 300 µl of whole EDTA blood was frozen to a solid at - 20° C, thawed at room temperature and distributed into three separate 8mm x 40mm glass Shell Vials. For the first vial, 400 µl of water was added (the no treatment sample). To the second vial was added 0.5 - 5U bovine plasminogen, 500 - 5,000 U streptokinase and 0.01 - 10 U human urokinase (Sigma Aldrich) in 400 µl water , (referred to as "plasmin" in Figs. 21-26). To the third vial was added 100 - 4,000 U Phospholipase A₂ (Sigma Aldrich) in 400 µl water. Each vial was gently mixed and incubated at room temperature for 2 minutes then exposed to the FCA Polarized Optical System described below and shown in FIG. 27.

The FCA Polarized Optical System was composed of appositionally placed lasers **10, 20** (e.g., Lasermate 785 nm Cat. # IML 785-800FLD and Lasermate 690 nm Cat. # RML690-800FLD lasers) each combined with polarizers **12** (e.g., Thor Labs nanoparticle linear polarizers Cat. # LPVIS050-MP) mounted on rotating mounts **14** (e.g., Thor Labs motorized precision rotation stages with servo drivers, Cat. # PRM1Z8E). The rotating mount was positioned so that a line transecting the 0° and 190° positions was parallel to the earth. A mirror **30** (e.g., Thor Labs knife edge right angle prism dielectric mirror, Cat. # MRAK25-E02 and Collimation Package Cat. # F810SMA-780) may be used to direct the laser output(s) into a series of collimators **40, 42** (e.g., Ocean Optics 84-series Collimating Lens) serving to shape the beam and appropriately illuminate the sample.

After passing through the collimators **40, 42,** the light was transmitted through the sample cuvette **60** and into another collimator **44** (e.g., Ocean Optics 84-series Collimating Lens), for further processing of the transmitted light. The collimator **44** was attached to a bifurcated optical fiber assembly **52** (e.g., Thor Labs Bifurcated Optical Fiber Assembly Cat. # Cat. # QBIF600-VIS-NIR) where light signal is supplied to separate photodiode power sensors (e.g., integrating sphere sensors from Thor Labs Cat. # S142C). Each power sensor was equipped with appropriate filters **70, 80** (e.g., Semrock Brightline filter FF01-689/23-12.5 for 690 nm wavelength light and Semrock MaxLine filter number LL01-785-12.5 for 785 nm wavelength light, respectively).

The polarizer for the 785 nm laser was set at 195°. The polarizer for the 690 nm laser was set at 355°. Ten seconds after sample introduction to the light beam, readings from a Thorlabs' PM200 Touch Screen Power and Energy Meter were recorded for the 785 nm and the 690 nm signals. The polarizer for the 690 nm laser was then rotated to the 170° setting and the signal was recorded after 10 seconds.

In FIGs. 21 - 26 are listed readings for various samples taken from various confirmed clinical cases. In the upper half of each figure are listed the actual energy readings in micro watts (µW). Below these data blocks are numbers that represent the differences between 1) plasmin and phospholipase, 2) no treatment and plasmin and 3) no treatment and phospholipase. For the 785 nm reading, the 195° designation describes the setting used to position the polarizer for that laser. For the 690 nm laser, readings were taken when the polarizer was positioned at either 170° or 355°. These readings were processed in order to derive a three position diagnostic code made up of +'s, -'s and 0's. A +, -, or 0 was assigned for each of the 785 nm 195° reading, the 690 nm 170° reading, and the 690nm 355° reading, which taken together indicated the presence or absence, and also the type of infection that a patient had.

For the 785 nm reading (the first position in the diagnostic code), the differences between 1) plasmin and phospholipase, 2) no treatment and plasmin and 3) no treatment and phospholipase were listed in that order from top to bottom and the direction that yielded a valid calculation was noted. For example, in FIG. 21, 15 + 5 = 20 while in the other direction 20 + 5 did not equal 15, hence the direction of the valid calculation was from bottom to top (see arrow). It was found that if the valid calculation proceeded from bottom to top, the patient from whom the blood was drawn did not have a viral or bacterial infection (FIG. 21). It was found that if the valid calculation proceeded downward (i.e., from top to bottom), the patient had a viral or bacterial infection (FIGs. 21-26). For example, in FIG. 21, 14 + -10 = 4 while in the other direction 4 + -10 does not equal 14, hence the direction of the valid calculation was from top to bottom (see arrow). If the valid calculation proceeded upward (bottom to top), a negative determination of "-" was recorded. If the valid calculation proceeded downward (top to bottom), a positive determination of "+" was recorded, unless two of the valid calculations at two wavelengths (that proceeded downward) resulted in negative numbers, in which case a "0" was recorded (see, e.g., FIG. 26, where 30 + -33 = -3 and 30 + -50 = -20, -3 and -20 are negative numbers, so a "0" was recorded).

For each of the two 690 nm readings the differences between 1) plasmin and phospholipase, 2) no treatment and plasmin and 3) no treatment and phospholipase were derived as with the 785 nm readings, but with the following modifications with regard to whether the value results from the differences was marked as positive or negative. When calculating the difference between plasmin and phospholipase treated samples, the resulting value was designated as negative if the phospholipase reading was lower than the plasmin reading. A zero difference was of course left as zero. When calculating the difference between the no treatment and plasmin treated samples the resulting value was designated as negative if the plasmin reading was lower than the no treatment reading (see, e.g., FIG. 25, where plasmin minus no treatment is -31 because 80 is lower than 111). When calculating the difference between the no treatment and phospholipase treated samples the resulting value was designated as negative if the phospholipase reading was lower than the no treatment reading (see, e.g., FIG. 26 where no treatment minus phospholipase is -20 because 80 is lower than 100).

For the second and third positions in the diagnostic code (the 690 nm readings at the 170° or 355° orientations of the polarizers), when the plasmin minus phospholipase number was negative a "-" designation was made in the second position of the diagnostic code. When two of the numbers are negative a "0" designation is made for the diagnostic code. If all three of the numbers are negative a "-" designation is made.

It was found that the - - - pattern was associated with a blood culture negative condition (FIG. 21), the + + + pattern was associated with a blood stream infection (FIG. 22), the + - 0 pattern was associated with a urinary tract infection (FIG. 23), the + 0 0 pattern was associated with bacterial pneumonia (FIG. 24), the + - - pattern was associated with a lower respiratory tract bacterial infection (FIG. 25), and the 0 0 0 pattern was associated with a systemic viral infection (FIG. 26).

These results demonstrate that treatment of blood with specific combinations of enzymes, followed by analysis of the treated blood using transmittance of polarized light at multiple wavelengths through the samples was able to not only identify patients as having or not having an infection, but also distinguish between different types of infection.

### Example 2: Microscopic analysis of Blood samples

Thin film microscopy was performed as follows. Blood from cases of infection or no infection were frozen and then treated with either water or enzymes as described previously in Example 1. Fifty microliters of sample were expressed on glass microscope slides and allowed to sit for 3 minutes followed by the addition of 50 ul of fixative. After 10 minutes of incubation the slides were immersed in a fixative bath for 10 minutes. Slides were then rinsed in distilled water for 1 minute. Slides were then immersed in Hematoxylin for 3 minutes, rinsed in running distilled water for 30 seconds, immersed in a bath of Scott's Tap Water solution for 5 minutes, rinsed in distilled water for 30 seconds and immersed in 95% ethanol for 1 minute. The slides were then immersed in Papanicolaou Stain OG 6-EA for 3 minutes, immersed in two sequential baths of 95% ethanol followed by immersion into two sequential baths of xylene. Mounting media and cover slips were applied and the slides viewed at 40 x using a standard light microscope.

When there was no infection, the phospholipase treated samples produced a granular background that impeded light transmission. When there was infection of the blood stream the phospholipase treated samples produced long linearly oriented aggregates with regular short branching patterns emanating from the sides of the central core fibers. When there was infection of an organ such as the bladder, the phospholipase treated samples produced larger numbers of smaller aggregates that are not as long compared to the aggregates found in blood stream infections.

When the subject had a bacterial infection that was not in the blood stream, the fibrin polymers/networks that became bound to red cell surfaces were smaller compared to those bound to red cell surfaces of patients who have a blood stream infection. These smaller fibrin networks appeared to provide enough occlusion (in conjunction with DNA) of phospholipid fragments. Plasmin was found to eliminate this effect.

The minimal complexity of the fibrin polymers in this cohort allow for more complete breakdown of phospholipids by phospholipase A₂.

The anisotropic molecular structure of fibrin enabled it to be seen with polarized light. Automated polarized light microscopy methods have been used to quantify structural parameters of fibrin (18).

### Example 3

Whole blood samples were taken from various confirmed clinical cases of various infection types and the anticoagulant EDTA was added. Each clinical case presented with one of the following: no infection (either blood culture negative or infection that was treated previously), *Actinomyces* bloodstream infection, *E. coli* bloodstream infection, *Neisseria meninitides* bloodstream infection, *K. pneumoniae* in bile and urine, bacterial pneumonia, cellulitis on foot, or falciparum malaria. 3 X 300 µl of whole EDTA blood was frozen to a solid at - 20° C, thawed at room temperature and distributed into a sample cartridge **200** containing 3 separate chambers for each sample. To the first chamber, 400 µl of water was added (the no treatment sample). To the second chamber was added 0.5 - 5U bovine plasminogen, 500 - 5,000 U streptokinase and 0.01 - 10 U human urokinase (Sigma Aldrich) in 400 µl water (referred to as "plasmin" in Figs. 1-13). To the third chamber was added 100 - 4,000 U Phospholipase A₂ (Sigma Aldrich) in 400 µl water. The samples were gently mixed and incubated at room temperature for 2 minutes then exposed to the FCA Polarized Optical System described below and shown in FIG. 17.

The FCA Polarized Optical System was composed of appositionally placed lasers **10, 20** (e.g., Lasermate 785 nm Cat. # IML 785-800FLD and Lasermate 690 nm Cat. # RML690-800FLD lasers) each combined with polarizers **12** (e.g., Thor Labs nanoparticle linear polarizers Cat. # LPVIS050-MP) mounted on rotating mounts **14** (e.g., Thor Labs motorized precision rotation stages with servo drivers, Cat. # PRM1Z8E). The rotating mount was positioned so that a line transecting the 0° and 180° positions was parallel to the earth. A mirror **30** (e.g., Thor Labs knife edge right angle prism dielectric mirror, Cat. # MRAK25-E02 and Collimation Package Cat. # F810SMA-780) was used to direct the laser output(s) and appropriately illuminate the sample.

The light was transmitted through the sample cartridge **200** held in place with a cartridge holder **300** and into a collimator **44** (e.g., Ocean Optics 84-series Collimating Lens), for further processing of the transmitted light. The collimator **44** was attached to a bifurcated optical fiber assembly **52** (e.g., Thor Labs Bifurcated Optical Fiber Assembly Cat. # Cat. # QBIF600-VIS-NIR) where light signal is supplied to separate photodiode power sensors (e.g., integrating sphere sensors from Thor Labs Cat. # S142C). Each power sensor was equipped with appropriate filters **70, 80** (e.g., Semrock Brightline filter FF01-689/23-12.5 for 690 nm wavelength light and Semrock MaxLine filter number LL01-785-12.5 for 785 nm wavelength light, respectively). The bifurcated optical fiber assembly was rotated such that one cable **54** was 7mm higher than the other cable **53** (see FIG. 19 and 20).

The polarizer for the 785 nm laser was set at 140°. The polarizer for the 690 nm laser was set at 80°. Ten seconds after sample introduction to the light beam, readings from a Thorlabs' PM200 Touch Screen Power and Energy Meter were recorded for the 785 nm and the 690 nm signals. The polarizer for the 690 nm laser was then rotated to the 95° setting and the signal was recorded after 10 seconds.

In FIGs. 1-13 are listed readings for various samples taken from various confirmed clinical cases. In the upper half of each figure are listed the actual energy readings in micro watts (µW). Below these data blocks are numbers that represent the differences between a) plasmin and phospholipase, b) no treatment and plasmin and c) no treatment and phospholipase. For the 785 nm reading, the 140° designation describes the setting used to position the polarizer for that laser. For the 690 nm laser, readings were taken when the polarizer was positioned at either 80° or 95°. These readings were processed in order to derive a diagnostic code which taken together indicated the presence or absence, and also the type of infection that a patient had.

As shown in Fig. 1, for rows A, B and C ((A) plasmin minus phospholipase, (B) no treatment minus plasmin and (C) no treatment minus phospholipase), when it is just the 'B' row numbers where all three are negative then fungal or bacterial blood stream infection was indicated. As shown in Fig. 2, for rows A, B and C, when it is just the 'B' row numbers where all three are negative then fungal or bacterial blood stream infection was indicated. As shown in Fig. 3, for rows A, B and C, when it is just the 'B' row numbers where all three are negative then fungal or bacterial blood stream infection was indicated. As shown in Fig. 4, for rows A, B and C when either 1) all numbers are positive or 2) there is at least one zero value in the 785 nm readings or 3) if at least all the 'A' row numbers are negative values then neither a bacterial nor fungal infection was indicated. As shown in Fig. 5, for rows A, B and C when either 1) all numbers are positive or 2) there is at least one zero value in the 785 nm readings or 3) if at least all the 'A' row numbers are negative values then neither a bacterial nor fungal infection was indicated. As shown in Fig. 6, for rows A, B and C when either 1) all numbers are positive or 2) there is at least one zero value in the 785 nm readings or 3) if at least all the 'A' row numbers are negative values then neither a bacterial nor fungal infection was indicated. As shown in Fig. 7, for rows A, B and C when either 1) all numbers are positive or 2) there is at least one zero value in the 785 nm readings or 3) if at least all the 'A' row numbers are negative values then neither a bacterial nor fungal infection was indicated. As shown in Fig. 8, for rows A, B and C when either 1) all numbers are positive or 2) there is at least one zero value in the 785 nm readings or 3) if at least all the 'A' row numbers are negative values then neither a bacterial nor fungal infection was indicated. As shown in Fig. 9, for rows A, B and C, when just row 'A' and row 'C' display all three numbers as being negative then an abdominal bacterial infection was indicated. For abdominal infections there may also be identical readings at both orientations of the 690 nm laser light polarizer for the no treatment sample. As shown in Fig. 10, for rows A, B and C, when just row 'A' and row 'C' display all three numbers as being negative then an abdominal bacterial infection is indicated. For abdominal infections there may be identical readings at both orientations of the 690 nm laser light polarizer for the no treatment sample. As shown in Fig. 11, for rows A, B and C when just the 'B' and 'C' rows display all three numbers as being negative then fungal or bacterial infection of the lungs was indicated. As shown in Fig. 12, for rows A, B and C when at least one number in just the 785 nm column is negative but the numbers in the 690nm columns are all positive then an unspecified bacterial or fungal infective process was indicated outside the blood stream. As shown in Fig. 13, for rows A, B and C when either 1) all numbers are positive or 2) there is at least one zero value in the 785 nm readings or 3) if at least all the 'A' row numbers are negative values then neither a bacterial nor fungal infection was indicated and a zero in the 785 nm calculations column that designates the sample as negative for bacterial infection supersedes any other rule.

These results demonstrate that treatment of blood with specific combinations of enzymes, followed by analysis of the treated blood using transmittance of polarized light at multiple wavelengths through the samples was able to not only identify patients as having or not having an infection, but also distinguish between different types of infection.

### Example 4.

### Exemplary tuning method

An exemplary method for tuning an optical system for detection of differences in anticoagulant-treated blood samples from people with infection versus without infection is next described. The optical system contains a bifurcated cable detection system (see, e.g., the bifurcated systems 100 and the bifurcated cable fiber assembly **52** shown in FIGs. 17-20 or FIG. 27), two lasers at two wavelengths (e.g., 785 and 690 nm) with polarizers mounted on rotating mounts, and a mirror to adjust the light from the laser onto the samples. These components can be in any configuration that results in transmittance of light through an anticoagulant-treated blood sample. The blood samples are frozen, thawed, and treated with enzymes as described in Example 1. A set of samples (no treatment, plasmin, phospholipase) from a subject without an infection and a set of samples from a subject with an infection are loaded into sample cartridges (see, e.g., FIGs. 14 and 15) or into cuvettes. The samples are then loaded into the optical system. The optical system is then tuned (e.g., by rotating the bifurcated cable, by rotating the sample, by rotating the polarizers, and/or by altering the angle of the lasers relative to the mirror) to maximize the differences in readings at the two wavelengths between the blood samples from the subject without an infection and from the subject with an infection.

For example, initial polarizer settings are chosen. The bifurcated cable can then be rotated such that the two cables within the bifurcated cable are not aligned with each other parallel to the earth (see, e.g., cables 53 and 54 within bifurcated cable fiber assembly 52 in FIGs. 19 and 20). The rotation is continued until the largest difference described above between the samples is observed. The sample is then rotated to achieve the highest transmission into the rotated cable assembly. The polarizers are then rotated to derive optimal polarizer positions for the rotated cable and samples.

### Description of the refraction events

Without wishing to be bound by theory, it is thought that the refraction events that are detected with the above system or with the systems in Examples 1 and 2 (which are measured by the transmittance of polarized light through the sample) are due to the use of the bifurcated optical fiber system. The cables within the optical fiber system each independently detect a single wavelength and will only detect that light if the light is refracted into the area of the cable, which is then sent to the detector. Without wishing to be bound by theory, the freezing, thawing, and different enzymes treatments of the blood change the refraction pattern of the blood, which change the angle of the light that emerges from the sample. The angle of the light determines whether the refracted light hits one or both of the cables and in turn is detected by one or both of the detectors (which can be exaggerated by rotating the cable system prior to the measurements). Thus, without wishing to be bound by theory, it is thought that the change in transmittance is due, at least in part, to these changes in refraction in the differently-processed blood samples. As a result, it is to be understood that it is the differences in transmission of light between the differently-treated blood samples (plasmin, phospholipase, no enzyme control) after freeze/thaw for each subject that identifies the subject's condition (infection, no infection, etc.). It is also to be understood that the values and algorithm derived for identifying patients will depend upon the differences in the optical system used. Examples 1 and 2 describe two exemplary optical systems, which used different optical set-ups and different angles of polarized light to arrive at diagnostic findings, both of which were valid for identifying subjects with or without infection.

### References

1) Mavrommatis AC. et al. Coagulation system and platelets are fully activated in uncomplicated sepsis. Crit Care Med. 2000;28:451-7.
2) Dixon B. The role of microvascular thrombosis in sepsis. Anaesth. Intensive Care. 2004;32:619-29
3) Dhainaut J-F, et al. Treatment effects of drotrecogin alfa (activated) in patients with severe sepsis with or without overt disseminated intravascular coagulation. J Thromb Haemost. 2004;2:1924-33
4) Schochl H. et al. Thromboelastometry (TEM®) Findings in Disseminated Intravascular Coagulation in a Pig Model of Endotoxinemia Mol Med. 2011 Mar-Apr; 17(3-4): 266-272
5) Levi M, van der Poll T. Inflammation and coagulation. Crit Care Med. 2010;38:S26-34.
6) Bakhtiari K. et al. Prospective validation of the International Society of Thrombosis and Haemostasis scoring system for disseminated intravascular coagulation. Crit Care Med. 2004;32:2416-21.
7) Hartert H. et al.Blutgerinnungsstudien mit der Thrombelastographie, einem neuen Untersuchungsvefahren. Klinische Wochenschrift 1948; 26:577-583
8) Rodelo J.R. et al. D-dimer is a significant prognostic factor in patients with suspected infection and sepsis. Am J Emerg Med. 2012 Nov;30(9):1991-9.
9) Wacker C. Procalcitonin as a diagnostic marker for sepsis: a systematic review and meta-analysis. Lancet Infect Dis. 2013 May;13(5):426-35.
10) Tsalik E.L. Discriminative value of inflammatory biomarkers for suspected sepsis. J Emerg Med. 2012 Jul;43(1):97-106
11) Semeraro N. Sepsis, thrombosis and organ dysfunction. Thromb Res. 2012 Mar;129(3):290-5.
12) Valerio-Rojas J.C. et al. Outcomes of severe sepsis and septic shock patients on chronic antiplatelet treatment: a historical cohort study. Crit Care Res Pract. 2013;2013:782573.
13) Rahman M. Platelet shedding of CD40L is regulated by matrix metalloproteinase-9 in abdominal sepsis. J Thromb Haemost. 2013 Apr 26.
14) Wei M. Depletion of neutrophil extracellular traps in vivo results in hypersusceptibility to polymicrobial sepsis in mice. Critical Care 2012, 16:R137.
15) Brzoska T. Binding of thrombin-activated platelets to a fibrin scaffold through α(IIb)β3 evokes phosphatidylserine exposure on their cell surface. PLoS One. 2013;8(2):e55466.
16) Guillaumin J. Assessment of a dimethyl sulfoxide-stabilized frozen canine platelet concentrate. Am J Vet Res. 2008 Dec;69(12):1580-6.
17) Wohner N. et al. Lytic resistance of fibrin containing red blood cells. Arterioscler Thromb Vasc Biol. 2011 Oct;31(10):2306-13.
18) Whittaker P. et al. Fibrin Architecture in Clots: A Quantitative Polarized Light Microscopy Analysis Blood Cells Mol Dis. 2009; 42(1): 51-56.

### OTHER EMBODIMENTS

Each feature disclosed in this specification may be replaced by an alternative feature serving the same, equivalent, or similar purpose. Thus, unless expressly stated otherwise, each feature disclosed is only an example of a generic series of equivalent or similar features.

From the above description, one skilled in the art can easily ascertain the essential characteristics of the present disclosure, and without departing from the scope thereof, can make various changes and modifications of the disclosure to adapt it to various usages and conditions. Thus, other embodiments are also within the claims.

### EQUIVALENTS

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of' or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of." "Consisting essentially of," when used in the claims, shall have its ordinary meaning as used in the field of patent law.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of' and "consisting essentially of' shall be closed or semi-closed transitional phrases.

## Claims

1. A method for assaying a blood sample obtained from a subject, the method comprising the steps of:
a) providing a first and a second anticoagulant-treated blood sample that have been previously frozen and thawed;
b) contacting the second anticoagulant-treated blood sample with:
i) plasmin or plasminogen and a plasminogen activator; or
ii) an enzyme that will break down a phospholipid bilayer;
c) passing light from an artificial source through the first anti-coagulant treated blood sample and through the second anti-coagulated treated blood sample after the contacting in step b);
d) measuring transmittance of the light through the first and second anti-coagulant treated blood samples; and
e) identifying the presence, absence and/or type of infection in the blood, if any, based on the transmittance of the light through the first anti-coagulant treated blood sample and the transmittance of the light through the second anti-coagulant treated blood sample.

2. The method of claim 1, wherein step: a) further comprises providing a third anticoagulant-treated blood sample that have been previously frozen and thawed; step b) comprises contacting the second anticoagulant-treated blood sample with plasmin or plasminogen and a plasminogen activator and further comprises contacting the third anticoagulant-treated blood sample with an enzyme that will break down a phospholipid bilayer; step c) further comprises passing light from an artificial source through the third anti-coagulant treated blood samples; step d) further comprises measuring transmittance of the light through the third anti-coagulant treated blood samples; and step e) further comprises identifying the presence, absence and/or type of infection in the blood, if any, based on the transmittance of the light through the third anti-coagulant treated blood sample.

3. The method of claim 2, wherein the method further comprises comparing the transmittance of the light through the first anticoagulant-treated blood sample with the transmittance of light through the second and third anticoagulant-treated blood samples.

4. The method of claim 2 or 3, wherein the method further comprises subtracting the transmittance of light through the second anticoagulant-treated blood sample from the transmittance of light through the first anticoagulant-treated blood sample and subtracting the transmittance of light through the third anticoagulant-treated blood sample from the transmittance of light through the first anticoagulant-treated blood sample.

5. The method of claim 4, wherein the method further comprises subtracting the transmittance of light through the third anticoagulant-treated blood sample from the transmittance of light through the second anticoagulant-treated blood sample, or subtracting the transmittance of light at each wavelength through the third anticoagulant-treated blood sample from the transmittance of light at each wavelength through the second anticoagulant-treated blood sample.

6. The method of any one of claims 1 to 4, wherein the plasminogen activator is streptokinase and/or urokinase, and/or wherein contacting with the plasminogen and the plasminogen activator comprises contacting with plasminogen, streptokinase, and urokinase.

7. The method of any one of claims 1 to 4, wherein contacting with the plasmin comprises producing plasmin by combining plasminogen, streptokinase, and urokinase and contacting the sample with the produced plasmin.

8. The method of any one of claims 1 to 7, wherein transmission of light is measured at a first and a second wavelength, and the identification of the presence, absence and/or type of infection in the blood, if any, is based on the transmittance of the light at the two wavelengths.

9. The method of any one of the preceding claims, wherein the first wavelength is between 775-795 nm and the second wavelength is between 680-700 nm.

10. The method of claim 9, wherein the first wavelength is 785 nm, and the second wavelength is 690 nm.

11. The method of claim 9 or 10, when dependent on claim 2, wherein the method further comprises comparing the transmittance of the light at the first wavelength and the second wavelength through the first anticoagulant-treated blood sample with the transmittance of light at the first wavelength and the second wavelength through the second and third anticoagulant-treated blood samples.

12. The method of claim 9 or 10 when dependent on claim 2, wherein the method further comprises subtracting the transmittance of light at each wavelength through the second anticoagulant-treated blood sample from the transmittance of light at each wavelength through the first anticoagulant-treated blood sample and subtracting the transmittance of light at each wavelength through the third anticoagulant-treated blood sample from the transmittance of light at each wavelength through the first anticoagulant-treated blood sample.

13. The method of any one of the preceding claims, wherein the light is polarized light and/or the light is measured using a photodiode power sensor.

14. The method of claim 13, wherein the light is measured using two photodiode sensors, and the light is passed through a bifurcated cable system prior to reaching the two photodiode power sensors.

15. The method of claim 1, 9, 10, 13, or 14, wherein the artificial source is a laser.

16. The method of any one of the preceding claims, wherein the enzyme that will break down a phospholipid bilayer is phospholipase A₂.

17. The method of any one of the preceding claims, wherein the first sample is contacted with water.

## Patentansprüche

1. Verfahren zum Testen einer von einem Patienten erhaltenen Blutprobe, wobei das Verfahren die folgenden Schritte umfasst:
a) Bereitstellen einer ersten und einer zweiten mit einem Antikoagulans behandelten Blutprobe, die zuvor eingefroren und aufgetaut wurden;
b) Inkontaktbringen der zweiten mit einem Antikoagulans behandelten Blutprobe mit:
i. Plasmin oder Plasminogen und einem Plasminogenaktivator; oder
ii. einem Enzym, das eine Phospholipid-Doppelschicht aufspaltet;
c) Durchleiten von Licht aus einer künstlichen Quelle durch die erste mit einem Antikoagulans behandelte Blutprobe und durch die zweite mit einem Antikoagulans behandelte Blutprobe nach dem Inkontaktbringen in Schritt b);
d) Messen der Lichtdurchlässigkeit durch die erste und zweite mit einem Antikoagulans behandelte Blutprobe; und
e) Identifizieren des Vorhandenseins, des Nichtvorhandenseins und/oder der Art der Infektion im Blut, falls vorhanden, basierend auf der Lichtdurchlässigkeit durch die erste mit einem Antikoagulans behandelte Blutprobe und der Lichtdurchlässigkeit durch die zweite mit einem Antikoagulans behandelte Blutprobe.

2. Verfahren nach Anspruch 1, wobei Schritt: a) weiterhin das Bereitstellen einer dritten mit einem Antikoagulans behandelten Blutprobe umfasst, die zuvor eingefroren und aufgetaut wurde;
wobei Schritt b) das Inkontaktbringen der zweiten mit einem Antikoagulans behandelten Blutprobe mit Plasmin oder Plasminogen und einem Plasminogenaktivator und ferner das Inkontaktbringen der dritten mit einem Antikoagulans behandelten Blutprobe mit einem Enzym umfasst, das eine Phospholipid-Doppelschicht aufspaltet;
wobei Schritt c) weiterhin das Leiten von Licht aus einer künstlichen Quelle durch die dritten mit einem Antikoagulans behandelten Blutproben umfasst;
wobei Schritt d) ferner das Messen der Lichtdurchlässigkeit durch die dritten mit einem Antikoagulans behandelten Blutproben umfasst; und
wobei Schritt e) ferner das Identifizieren des Vorhandenseins, des Nichtvorhandenseins und/oder der Art der Infektion im Blut, falls vorhanden, auf der Grundlage der Lichtdurchlässigkeit durch die dritte mit einem Antikoagulans behandelte Blutprobe umfasst.

3. Verfahren nach Anspruch 2, wobei das Verfahren ferner das Vergleichen der Lichtdurchlässigkeit durch die erste mit einem Antikoagulans behandelte Blutprobe mit der Lichtdurchlässigkeit durch die zweite und dritte mit einem Antikoagulans behandelte Blutprobe umfasst.

4. Verfahren nach Anspruch 2 oder 3, wobei das Verfahren ferner das Subtrahieren der Lichtdurchlässigkeit durch die zweite mit einem Antikoagulans behandelte Blutprobe von der Lichtdurchlässigkeit durch die erste mit einem Antikoagulans behandelte Blutprobe und das Subtrahieren der Lichtdurchlässigkeit durch die dritte mit einem Antikoagulans behandelte Blutprobe von der Lichtdurchlässigkeit durch die erste mit einem Antikoagulans behandelte Blutprobe umfasst.

5. Verfahren nach Anspruch 4, wobei das Verfahren ferner das Subtrahieren der Lichtdurchlässigkeit durch die dritte mit einem Antikoagulans behandelte Blutprobe von der Lichtdurchlässigkeit durch die zweite mit einem Antikoagulans behandelte Blutprobe oder das Subtrahieren der Lichtdurchlässigkeit bei jeder Wellenlänge durch die dritte mit einem Antikoagulans behandelte Blutprobe von der Lichtdurchlässigkeit bei jeder Wellenlänge durch die zweite mit einem Antikoagulans behandelte Blutprobe umfasst.

6. Verfahren nach einem der Ansprüche 1 bis 4, wobei der Plasminogenaktivator Streptokinase und/oder Urokinase ist und/oder wobei das Inkontaktbringen mit dem Plasminogen und dem Plasminogenaktivator das Inkontaktbringen mit Plasminogen, Streptokinase und Urokinase umfasst.

7. Verfahren nach einem der Ansprüche 1 bis 4, wobei das Inkontaktbringen mit dem Plasmin das Herstellen von Plasmin durch Kombinieren von Plasminogen, Streptokinase und Urokinase sowie das Inkontaktbringen der Probe mit dem hergestellten Plasmin umfasst.

8. Verfahren nach einem der Ansprüche 1 bis 7, wobei die Lichtübertragung bei einer ersten und einer zweiten Wellenlänge gemessen wird und die Identifizierung des Vorhandenseins, Nichtvorhandenseins und/oder der Art der Infektion im Blut, falls vorhanden, auf der Durchlässigkeit des Lichts bei den beiden Wellenlängen beruht.

9. Verfahren nach einem der vorstehenden Ansprüche, wobei die erste Wellenlänge zwischen 775-795 nm und die zweite Wellenlänge zwischen 680-700 nm liegt.

10. Verfahren nach Anspruch 9, wobei die erste Wellenlänge 785 nm und die zweite Wellenlänge 690 nm beträgt.

11. Verfahren nach Anspruch 9 oder 10, wenn abhängig von Anspruch 2, wobei das Verfahren ferner das Vergleichen der Durchlässigkeit des Lichts bei der ersten Wellenlänge und der zweiten Wellenlänge durch die erste mit einem Antikoagulans behandelte Blutprobe mit der Durchlässigkeit des Lichts bei der ersten Wellenlänge und der zweiten Wellenlänge durch die zweite und dritte mit einem Antikoagulans behandelte Blutprobe umfasst.

12. Verfahren nach Anspruch 9 oder 10, wenn von Anspruch 2 abhängig, wobei das Verfahren ferner das Subtrahieren der Durchlässigkeit von Licht bei jeder Wellenlänge durch die zweite mit einem Antikoagulans behandelte Blutprobe von der Durchlässigkeit von Licht bei jeder Wellenlänge durch die erste mit einem Antikoagulans behandelte Blutprobe und das Subtrahieren der Durchlässigkeit von Licht bei jeder Wellenlänge durch die dritte mit einem Antikoagulans behandelte Blutprobe von der Durchlässigkeit von Licht bei jeder Wellenlänge durch die erste mit einem Antikoagulans behandelte Blutprobe umfasst.

13. Verfahren nach einem der vorhergehenden Ansprüche, wobei das Licht polarisiertes Licht ist und/oder wobei das Licht mit Hilfe eines Photodiodenleistungssensors gemessen wird.

14. Verfahren nach Anspruch 13, wobei das Licht mit Hilfe von zwei Photodiodensensoren gemessen wird und wobei das Licht durch ein gegabeltes Kabelsystem geleitet wird, bevor es die beiden Photodiodenleistungssensoren erreicht.

15. Verfahren nach Anspruch 1, 9, 10, 13 oder 14, wobei die künstliche Quelle ein Laser ist.

16. Verfahren nach einem der vorhergehenden Ansprüche, wobei das Enzym, das eine Phospholipid-Doppelschicht aufspaltet, Phospholipase A₂ ist.

17. Verfahren nach einem der vorhergehenden Ansprüche, wobei die erste Probe mit Wasser in Kontakt gebracht wird.

## Revendications

1. Procédé de dosage d'un échantillon de sang obtenu à partir d'un sujet, le procédé comprenant les étapes suivantes :
a) la fourniture d'un premier et d'un deuxième échantillon de sang traité par anticoagulant qui ont été préalablement congelés et décongelés ;
b) la mise en contact du deuxième échantillon de sang traité par anticoagulant avec :
i. de la plasmine ou du plasminogène et un activateur du plasminogène ; ou
ii. une enzyme qui décomposera une bicouche phospholipidique ;
c) le passage de la lumière d'une source artificielle à travers le premier échantillon de sang traité par anticoagulant et à travers le deuxième échantillon de sang traité par anticoagulant après la mise en contact à l'étape b) ;
d) la mesure de la transmittance de la lumière à travers les premier et deuxième échantillons de sang traités par anticoagulant ; et
e) l'identification de la présence, de l'absence et/ou du type d'infection dans le sang, le cas échéant, sur la base de la transmittance de la lumière à travers le premier échantillon de sang traité par anticoagulant et de la transmittance de la lumière à travers le deuxième échantillon de sang traité par anticoagulant.

2. Procédé selon la revendication 1, selon lequel l'étape : a) comprend en outre la fourniture d'un troisième échantillon de sang traité par anticoagulant qui a été préalablement congelé et décongelé ;
l'étape b) comprend la mise en contact du deuxième échantillon de sang traité par anticoagulant avec de la plasmine ou du plasminogène et un activateur du plasminogène et comprend en outre la mise en contact du troisième échantillon de sang traité par anticoagulant avec une enzyme qui décomposera une bicouche phospholipidique ;
l'étape c) comprend en outre le passage de la lumière d'une source artificielle à travers le troisième échantillon de sang traité par anticoagulant ;
l'étape d) comprend en outre la mesure de la transmittance de la lumière à travers le troisième échantillon de sang traité par anticoagulant ; et
l'étape e) comprend en outre l'identification de la présence, de l'absence et/ou du type d'infection dans le sang, le cas échéant, sur la base de la transmittance de la lumière à travers le troisième échantillon de sang traité par anticoagulant.

3. Procédé selon la revendication 2, le procédé comprenant en outre la comparaison de la transmittance de la lumière à travers le premier échantillon de sang traité par anticoagulant avec la transmittance de la lumière à travers les deuxième et troisième échantillons de sang traités par anticoagulant.

4. Procédé selon la revendication 2 ou 3, le procédé comprenant en outre la soustraction de la transmittance de la lumière à travers le deuxième échantillon de sang traité par anticoagulant de la transmittance de la lumière à travers le premier échantillon de sang traité par anticoagulant et la soustraction de la transmittance de la lumière à travers le troisième échantillon de sang traité par anticoagulant de la transmittance de la lumière à travers le premier échantillon de sang traité par anticoagulant.

5. Procédé selon la revendication 4, le procédé comprenant en outre la soustraction de la transmittance de la lumière à travers le troisième échantillon de sang traité par anticoagulant de la transmittance de la lumière à travers le deuxième échantillon de sang traité par anticoagulant, ou la soustraction de la transmittance de la lumière au niveau de chaque longueur d'onde à travers le troisième échantillon de sang traité par anticoagulant de la transmittance de la lumière au niveau de chaque longueur d'onde à travers le deuxième échantillon de sang traité par anticoagulant.

6. Procédé selon l'une quelconque des revendications 1 à 4, selon lequel l'activateur du plasminogène est la streptokinase et/ou l'urokinase, et/ou selon lequel la mise en contact avec le plasminogène et l'activateur du plasminogène comprend la mise en contact avec le plasminogène, la streptokinase et l'urokinase.

7. Procédé selon l'une quelconque des revendications 1 à 4, selon lequel la mise en contact avec la plasmine comprend la production de plasmine en combinant le plasminogène, la streptokinase et l'urokinase et la mise en contact de l'échantillon avec la plasmine produite.

8. Procédé selon l'une quelconque des revendications 1 à 7, selon lequel la transmittance de la lumière est mesurée au niveau d'une première et d'une seconde longueur d'onde, et l'identification de la présence, de l'absence et/ou du type d'infection dans le sang, le cas échéant, est basée sur la transmittance de la lumière au niveau des deux longueurs d'onde.

9. Procédé selon l'une quelconque des revendications précédentes, selon lequel la première longueur d'onde est comprise entre 775 et 795 nm et la seconde longueur d'onde est comprise entre 680 et 700 nm.

10. Procédé selon la revendication 9, selon lequel la première longueur d'onde est de 785 nm et la seconde longueur d'onde est de 690 nm.

11. Procédé selon la revendication 9 ou 10, lorsqu'elle dépend de la revendication 2, le procédé comprenant en outre la comparaison de la transmittance de la lumière au niveau de la première longueur d'onde et au niveau de la seconde longueur d'onde à travers le premier échantillon de sang traité par anticoagulant avec la transmittance de la lumière au niveau de la première longueur d'onde et au niveau de la seconde longueur d'onde à travers les deuxième et troisième échantillons de sang traités par anticoagulant.

12. Procédé selon la revendication 9 ou 10 lorsqu'elle dépend de la revendication 2, le procédé comprenant en outre la soustraction de la transmittance de la lumière au niveau de chaque longueur d'onde à travers le deuxième échantillon de sang traité par anticoagulant de la transmittance de la lumière au niveau de chaque longueur d'onde à travers le premier échantillon de sang traité par anticoagulant et la soustraction de la transmittance de la lumière au niveau de chaque longueur d'onde à travers le troisième échantillon de sang traité par anticoagulant de la transmittance de la lumière au niveau de chaque longueur d'onde à travers le premier échantillon de sang traité par anticoagulant.

13. Procédé selon l'une quelconque des revendications précédentes, selon lequel la lumière est une lumière polarisée et/ou la lumière est mesurée à l'aide d'un capteur de puissance à photodiode.

14. Procédé selon la revendication 13, selon lequel la lumière est mesurée à l'aide de deux capteurs à photodiode, et la lumière est passée à travers un système de câble bifurqué avant d'atteindre les deux capteurs de puissance à photodiode.

15. Procédé selon la revendication 1, 9, 10, 13 ou 14, selon lequel la source artificielle est un laser.

16. Procédé selon l'une quelconque des revendications précédentes, selon lequel l'enzyme qui décomposera une bicouche phospholipidique est la phospholipase A₂.

17. Procédé selon l'une quelconque des revendications précédentes, selon lequel le premier échantillon est mis en contact avec de l'eau.
